# EUROPÄISCHE PATENTSCHRIFT

(11) **EP 1 881 956 B1**
(45) Veröffentlichungstag und Bekanntmachung des Hinweises auf die Patenterteilung: **13.05.2009**
(21) Anmeldenummer: 06776325.0
(22) Anmeldetag: 20.07.2006
(51) Int. Cl.: C07C 215/64

(54) **3-(2-(DIMETHYLAMINO)METHYL-(CYCLOHEX-1-YL))PHENOL-MALEAT UND DESSEN KRISTALLINE FORMEN**
3-(2-(DIMETHYLAMINO)METHYL(CYCLOHEX-1-YL))PHENOL MALEATE AND ITS CRYSTALLINE FORMS
3-(2-(DIMETHYLAMINO)METHYL-(CYCLOHEX-1-YL))PHENOL-MALEAT ET SES FORMES CRISTALLINES

(30) Priorität: 22.07.2005 DE 102005034974
(43) Veröffentlichungstag der Anmeldung: 30.01.2008
(73) Patentinhaber: Grünenthal GmbH, 52078 Aachen (DE)
(72) Erfinder: SZELAGIEWICZ, Martin, 4142 Münchenstein (CH); HELL, Wolfgang, 52066 Aachen (DE); VON RAUMER, Markus, 4144 Arlesheim (CH); DE PAUL, Susan, Margaret, 8057 Zürich (DE); BERGHAUSEN, Jörg, CH-79541 Lörrach (CH); GRUSS, Michael, DE-52080 Aachen (DE)
(86) Internationale Anmeldenummer: PCT/EP2006/007160
(87) Internationale Veröffentlichungsnummer: WO 2007/009792

(56) Entgegenhaltungen:
- EP-A1- 0 753 506

## Beschreibung

Die vorliegende. Erfindung betrifft Salze von Maleinsäure und 3-[2-(Dimethylamino)methyl-(cyclohex-1-yl)]-phenol gemäß formel I , stabile kristalline Formen des Salzes sowie Verfahren zu deren Herstellung, eine pharmazeutische Zusammensetzung und die Verwendung des Salzes als pharmazeutischer Wirkstoff in einer Zusammensetzung.

In der EP-A1-0 753 506 werden 3-[2-(Dimethylamino)methyl-(cyclohex-1-yl)]-phenole mit analgetischer Wirkung beschrieben. Im Text wird erwähnt, dass man auch Salze aus den freien Basen herstellen kann, wobei aber Maleinsäure als mögliches Anion nicht enrvähnt wird. In den Beispielen werden ausschliesslich Hydrochloride bereitgestellt, also ein Salz mit monovalentem Anion. Die EP-A9-0 753 506 enthält keinerlei Hinweise, in welcher Stöchiometrie 3-[2-(Dimethylamino)methyl-(cyclohex-1-yl)]-phenole mit bivalenten Anionen vorliegen können, zum Beispiel als Hemi- oder 1:1-Salze. Die nähere Untersuchung von 3-[2-(Dimethylamino)methyl-(cyclohex-1-yl)]-phenol-hydrochlorid hat ergeben, dass sich diese kristalline Festsubstanz durch einen ausgeprägten Polymorphismus auszeichnet und mehrere kristalline, auch metastabile Formen bildet. Zusätzlich neigt dieses Hydrochlorid stark zur Bildung von Hydraten und Solvaten, was für die gezielte Herstellung einer spezifischen kristallinen Form ein erheblicher Nachteil ist. Das kristalline 3-[2-(Dimethylamino)methyl-(cyclohex-1-yl)]-phenol-hydrochtorid hat sich zudem als ausgesprochen hygroskopisch erwiesen. Dieses Eigenschaftsprofil von 3-[2-(Dimethylamino)methyl-(cyclohex-1-yl)]-phenol-hydrochlorid macht deutlich, dass es sehr schwierig ist, mit dieser Wirksubstanz pharmazeutische Zusammensetzungen mit reproduzierbaren Eigenschaften bereitzustellen, die auch Ober den Zeitraum einer Lagerung erhalten bleiben. Für die Erreichung dieser Ziele wären zumindest aufwendige Schutzmassnahmen erforderlich.

Es wurde nun überraschend gefunden, dass 3-[2-(Dimethylamino)methyl-(cyclohex-1-yl)]-phenol mit Maleinsäure ein Maleat als kristalline Festsubstanz bildet, bevorzugt in einer Zusammensetzung im Verhältnis 1:1 von Maleinsäure-Anion und 3-[2-(Dimethylamino)methyl-(cyclohex-1-yl)]-pbenol. Es wurde ferner überraschend gefunden, dass das Maleat nicht hygroskopisch ist, an Luft stabil ist und keine Hydrate beziehungsweise Solvate bildet. Es wurde auch überraschend gefunden, dass 3-[2-(Dimethylamino)methyl-(cyclohex-1-yl)]-phenol-maleat nur wenige, nämlich zwei kristalline, bei niedriger beziehungsweise bei höherer Temperatur stabile Formen zu bilden vermag, und dass sich auffindbare metastabile Formen in die bei Raumtemperatur stabile Form A umwandeln. Die bei höherer Temperatur stabile Form B kann ebenfalls in Form A umgewandelt werden, wobei beide Formen bei niedriger Temperatur in Mischung vorliegen können. Die kristalline Form A zeichnet sich auch durch eine hohe chemische Beständigkeit bei Temperaturen unter 100 °C aus. Ferner weist 3-[2-(Dimethylamino)methyl-(cyclohex-1-yl)]-phenol-maleat auch wertvolle biologische Eigenschaften, wie zum Beispiel gute Löslichkeit besonders in polaren und protischen Lösungsmitteln einschliesslich Wasser, und eine gute Bioverfügbarkeit auf. 3-[2-(Dimethylamino)methyl-(cyclohex-1-yl)]-phenol-maleat eignet sich auf Grund seines Eigenschaftsprofils hervorragend zur Formulierung von pharmazeutischen Zusammensetzungen.

Ein erster Gegenstand der Erfindung sind Salze der Maleinsäure mit 3-[2-(Dimethylamino)methyl-(cyclohex-1-yl)]-phenol der Formel I

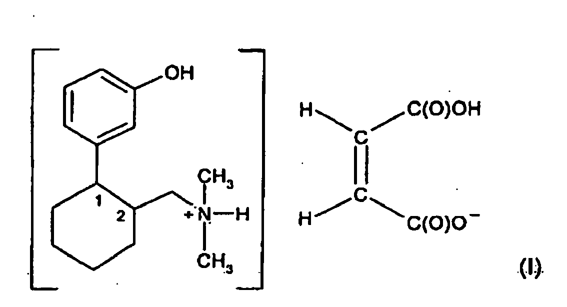

Die erfindungsgemäßen Verbindungen wie die Verbindungen der Formel I enthalten in den 1- und 2-Positionen des Cyclohexanringes je ein chirales C-Atom. Die erfindungsgemäßen Verbindungen wie die Verbindungen der Formel I umfassen alle Stereoisomeren und Gemische von Stereoisomeren. Bevorzugt sind Diastereomere oder Gemische von enantiomeren Diastereomeren mit Transkonfiguration des Phenylringes und der Dimethylaminomethylgruppe (1R,2R- beziehungsweise 1S,2S-Konfiguration), wobei das Enantiomer mit der absoluten Konfiguration (1R,2R) ganz besonders bevorzugt ist.
Die Struktur des (1R,2R)-Enantiomers von 3-[2-(Dimethylamino)methyl-(cyclohex-1-yl)]-phenol ist nachstehend wiedergegeben:

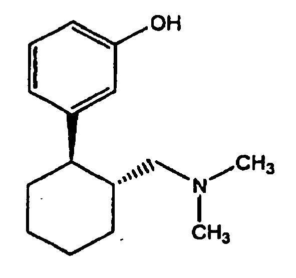

Die Verbindungen der Formel I können, analog zu den in der EP-A1-0 753 506 allgemein für die Herstellung von Salzen beschriebenen Verfahren, aus der freien Base und Umsetzung mit Maleinsäure oder Maleinsäureanhydrid in Gegenwart von Wasser erhalten werden. Die freie Base 3-[2-(Dimethylamino)methyl-(cyclohex-1-yl)]-phenol kann zum Beispiel aus dem in den Beispielen 9 und 10 der EP-A1-0 753 506 beschriebenen Hydrochlorid isoliert werden. Hierzu löst man das Hydrochlorid in einem organischen Lösungsmittel, versetzt mit einer wässrigen anorganischen Base, zum Beispiel Alkalimetallbasen oder auch Alkalimetallhydrogencarbonaten (wie LiOH, NaOH, KOH, NaHCO₃ und KHCO₃), und trennt die organische Phase ab. Die organische Phase kann getrocknet werden und entweder die Base in üblicher Weise isoliert oder gegebenenfalls nach einer Konzentrierung durch Verdampfen vom Lösungsmittel direkt zur Salzbildung verwendet werden.

Es wurde überraschend gefunden, dass die Salzbildung dann zu nur einer kristallinen Form führt, wenn man die Temperatur kontrolliert und keine Temperaturen von über 100 °C anwendet.

Ein weiterer Gegenstand der Erfindung ist ein Verfahren zur Herstellung von 3-[2-(Dimethylamino)methyl-(cyclohex-1-yl)]-phenol-maleat der Formel I, umfassend die Vereinigung von 3-[2-(Dimethylamino)methyl-(cyclohex-1-yl)]-phenol und Maleinsäure, wobei vorzugsweise wenigstens eine der Komponenten in gelöster oder suspendierter Form vorliegt.

Ein weiterer Gegenstand der Erfindung ist ein Verfahren zur Herstellung von 3-[2-(Dimethylamino)methyl-(cyclohex-1-yl)]-phenol-maleat der Formel I, umfassend die Schritte
a) Lösen oder Suspendieren von 3-[2-(Dimethylamino)methyl-(cyclohex-1-yl)]-phenol in einem Lösungsmittel,
b) Vermischen der Lösung oder Suspension mit Maleinsäure oder einer Lösung von Maleinsäure, und
c) Isolieren der Verbindung der Formel I,
wobei vorzugsweise in keiner Verfahrensstufe die Temperatur über 100 °C liegt und die Schritte a) und b) auch vertauscht werden können.

Die Temperatur beträgt bevorzugt nicht mehr als 80 °C, bevorzugter nicht mehr als 70 °C und besonders bevorzugt nicht mehr als 60 °C. Die Temperatur liegt beim Lösen gemäss Verfahrensstufe a) im allgemeinen höher als beim Vermischen gemäss Verfahrensstufe b).

Die freie Base und Maleinsäure können im molaren Verhältnis von 1:1 eingesetzt werden, oder Maleinsäure kann auch im Überschuss verwendet werden, zum Beispiel in einem molaren Verhältnis von bis zu 1,3, bevorzugt bis zu 1,1. Bei Verwendung eines Überschusses der freien Base werden keine Hemimaleate gebildet, selbst wenn man ein molares Verhältnis von Base zu Maleinsäure von 2:1 einstellt.

Die Menge von 3-[2-(Dimethylamino)methyl-(cyclohex-1-yl)]-phenol in der Lösung kann zum Beispiel 5 bis 70 Gew.-%, bevorzugt 10 bis 60 Gew.-%, bevorzugter 10 bis 50 Gew.-%, und besonders bevorzugt 15 bis 40 Gew.-% betragen, bezogen auf die Lösung. Die Lösung von 3-[2-(Dimethylamino)methyl-(cyclohex-1-yl)]-phenol kann erwärmt und anschliessend gegebenenfalls auf die Temperatur abgekühlt werden, die für das Vermischen mit Maleinsäure gewünscht ist.

In den hierin beschriebenen Verfahren zur Herstellung von 3-[2-(Dimethylamino)methyl-(cyclohex-1-yl)]-phenol-rnaleat kann die Maleinsäure jeweils auch in Form ihres Anhydrids eingesetzt werden.
Inerte (kompatible) Lösungsmittel für 3-[2-(Dimethylamino)methyl-(cyclohex-1-yl)]-phenol und Maleinsäure bzw. Maleinsäureanhydrid sind zum Beispiel aliphatische, cycloaliphatische und aromatische Kohlenwasserstoffe (Hexan, Heptan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutylether, t-Butylmethylether, Ethylenglykoldimethylether, Ethylenglykoldiethylether, Diethylenglykoldimethylether, Tetrahydrofuran, Dioxan), Ketone (Aceton, 2-Butanon, Methylisobutylketon), Carbonsäureester und Lactone (Essigsäureethyl- oder - methylester, Valerolacton), N-substituierte Lactame (N-Methylpyrrolidon), Carbonsäureamide (Dimethylacetamid, Dimethylformamid), acyclische Harnstoffe (Dimethylimidazolin), und Sulfoxide und Sulfone (Dimethylsulfoxid, Dimethylsulfon, Tetramethylensulfoxid, Tetramethylensulfon) und Alkohole (Methanol, Ethanol, 1-oder 2-Propanol, n-, i- und t-Butanol, 1-Pentanol, 1-Hexanol, 1-Heptanol, 1-Octanol, Ethylenglykolmonomethylether, Ethylenglykolmonoethylether, Diethylenglykolmonomethyl- oder monoethylether) und Wasser. Die Lösungsmittel können alleine oder in Mischung von wenigstens zwei Lösungsmitteln verwendet werden. Vorteilhaft verwendet man physiologisch unbedenkliche Lösungsmittel, die dem Fachmann geläufig sind.

Das Vermischen in der Verfahrensstufe b) kann mittels langsamer oder schneller Zugabe von einer Lösung zur anderen Lösung erfolgen. Eine oder beide Lösungen können erwärmt sein. Das Vermischen kann aber auch so vorgenommen werden, dass eine oder beide Lösungen sich bei Raumtemperatur befinden oder gekühlt sind, zum Beispiel bis auf -20 °C und bevorzugter bis auf -10 bis +10 °C, außerst bevorzugt -5 bis +5 °C. Nach dem Vermischen kann erwärmt und wieder gekühlt und es kann für einen bestimmten Zeitraum weitergerührt werden. Eine Kristallbildung kann auch durch Animpfen gefördert werden.

In der Regel bildet sich schon während des Mischens ein weisser Niederschlag, der kristallin und gut filtrierbar ist. Die Isolierung kann durch Dekantieren, Filtrieren oder Zentrifugieren erfolgen. Der kristalline Rückstand kann dann noch getrocknet werden, zum Beispiel mittels Erwärmen, Vakuumtrocknung oder Erwärmen im Vakuum, oder mittels eines gegebenenfalls erwärmten und inerten Gasstroms (Luft, Stickstoff, Edelgase). Die Verbindungen der Formel I werden in hohen Ausbeuten und hoher Reinheit erhalten. In der Regel sind keine oder nur wenige weitere Reinigungsschritte wie zum Beispiel Umkristallisationen erforderlich und das Produkt kann direkt zur Herstellung von pharmazeutischen Zusammensetzungen verwendet werden.
Ein weiterer Gegenstand der vorliegenden Erfindung ist ein Salz von Maleinsäure und 3-[2-(Dimethylamino)methyl-(cyclohex-1-yl)]-phenol der Formel I, erhältlich nach einem der vorstehend beschriebenen Verfahren.

Die Verbindungen der Formel I und das erfindungsgemässe Herstellungsverfahren bieten gegenüber entsprechenden Hydrochloriden erhebliche und teilweise unerwartete Vorteile. Da sich keine Hydrate und Solvate charakterisieren liessen, ist die Auswahl an einsetzbaren Lösungsmitteln breit und unkritisch. Die Stabilität an Luft und gegen Feuchtigkeit erlaubt ein offenes Handhaben ohne besondere Schutzmassnahmen. Die spontane Salzbildung und Bildung von kristallinen Niederschlägen sowie deren gute Filtrierbarkeit erlauben Verfahren im industriellen Massstab.

Die Verbindungen der Formel I werden beim erfindungsgemässen Verfahren der Salzbildung als kristalliner Festkörper überwiegend in einer polymorphen Form erhalten, die nachfolgend als Form A bezeichnet wird. Amorphe Formen der Verbindungen der Formel I sind einfach zum Beispiel mittels Gefriertrocknung beziehungsweise schnellem Abkühlen von Lösungen erhältlich. Amorphe Verbindungen der Formel I sind nicht sehr stabil und neigen in Gegenwart von Feuchtigkeit zur Kristallisation. Sie eignen sich daher besonders als Ausgangsmaterial zur gezielten Herstellung von kristallinen Formen.

Es wurde gefunden, dass die Verbindungen der Formel I als kristalline Festkörper polymorphe Formen bilden, die gezielt aus 3-[2-(Dimethylamino)methyl-(cyclohex-1-yl)]-phenol-maleat herstellbar sind und auf Grund ihrer Stabilität besonders als Wirkstoff zur Formulierung von pharmazeutischen Zusammensetzungen geeignet sind. Es ist bekannt [siehe zum Beispiel Z. Jane Li et al. in J. Pharm. Sci., Vol. 88(3), Seiten 337 bis 346 (1999)], dass Enantiomere identische Röntgen-Diffraktogramme und Raman-Spektren ergeben und somit gleiche polymorphe Formen bilden. Im Rahmen der Erfindung werden somit polymorphe Formen von allen Enantiomeren umfasst.

Ein weiterer Gegenstand der Erfindung ist eine kristalline Form von 3-[2-(Dimethylamino)methyl-(cyclohex-1-yl)]-phenol-maleat der Formel I, die ein charakteristisches Röntgen-Beugungsbild im Bereich von 2° bis 35° 20 mit ausgeprägten charakteristischen Linien aufweist, ausgedrückt in d-Werten (Å): 9.4 (vs), 6.8 (m), 5.56 (s), 5.30 (s), 5.22 (s), 4.71 (s), 4.66 (s), 4.24 (m), 4.12 (m), 4.03 (m), 3.98 (s) 3.76 (m), 3.27 (m); nachfolgend als Form A bezeichnet.

Zuvor und nachfolgend bedeuten die Abkürzungen in Klammern: (vs) = sehr starke Intensität, (s) = starke Intensität, (m) = mittlere Intensität, (w) = schwache Intensität, und (vw) = sehr schwache Intensität.

Ein weiterer Gegenstand der Erfindung ist eine kristalline Form A von 3-[2-(Dimethylamino)methyl-(cyclohex-1-yl)]-phenol-maleat der Formel I, die ein charakteristisches Röntgen-Beugungsbild mit den nachstehenden ausgeprägten Reflexen aufweist.

| **2Theta** | **rel. Intensität** |
|---|---|
| 9,38 | 100 |
| 9,94 | 5 |
| 10,35 | 8 |
| 12,76 | 6 |
| 13,07 | 20 |
| 13,49 | 7 |
| 15,94 | 43 |
| 16,72 | 52 |
| 16,98 | 40 |
| 17,54 | 15 |
| 18,86 | 61 |
| 19,04 | 48 |
| 19,28 | 8 |
| 20,01 | 5 |
| 20,57 | 6 |
| 20,96 | 24 |
| 21,57 | 23 |
| 22,03 | 20 |
| 22,33 | 30 |
| 23,69 | 15 |
| 24,84 | 8 |
| 25,01 | 9 |
| 25,71 | 11 |
| 26,35 | 7 |
| 26,75 | 13 |
| 27,24 | 16 |
| 27,66 | 9 |
| 27,95 | 6 |
| 28,40 | 5 |
| 28,68 | 9 |
| 29,87 | 6 |
| 30,76 | 8 |
| 31,37 | 4 |
| 31,67 | 4 |
| 31,99 | 6 |
| 32,53 | 4 |
| 32,80 | 5 |
| 33,55 | 7 |
| 35,20 | 5 |
| 36,93 | 5 |
| 37,58 | 5 |
| 44, 54 | 6 |
| 45,70 | 4 |

In der vorstehenden Tabelle sind die Peaklagen (in 2 Theta) sowie die relativen Intensitäten der Peaks angegeben. Der intensivste Peak ist dabei auf eine relative Intensität von 100 normiert.

Gegenstand der Anmeldung ist weiterhin eine kristalline Form A des 3-[2-(Dimethylamino)methyl-(cyclohex-1-yl)]-phenol-maleats der Formel I (Polymorph A), charakterisiert durch ein Pulverdiffraktogramm umfassend einen oder mehrere der folgenden Reflexe: 9,38 (100), 9,94 (5) und 10,35 (8) (jeweils ± 0,5 in 2 Theta, relative Intensität in Klammern). Vorzugsweise kann das Pulverdiagramm zusätzlich einen oder mehrere der folgenden Reflexe aufweisen: 12,76 (6), 15,94 (43), 17,54 (15), 19,28 (8), 28,68 (9) und 31,99 (6) (jeweils ± 0,2 in 2 Theta, relative Intensität in Klammern).

Ein anderer Gegenstand der Erfindung ist eine kristalline Form A von 3-[2-(Dimethylamino)methyl-(cyclohex-1-yl)]-phenol-maleat der Formel I, die ein charakteristisches Raman Spektrum mit charakteristischen Linien aufweist, ausgedrückt in Wellenzahlen (cm-1):

| Position (cm-1) | Intensität |
|---|---|
| 3060 | m |
| 3040 | m |
| 3021 | m |
| 2986 | m |
| 2966 | s |
| 2933 | s |
| 2920 | s |
| 2895 | m |
| 2879 | m |
| 2856 | s |
| 2812 | w |
| 1690 | m |
| 1616 | m |
| 1601 | m |
| 1569 | vw |
| 1467 | m |
| 1443 | m |
| 1412 | w |
| 1389 | m |
| 1361 | w |
| 1351 | m |
| 1332 | w |
| 1322 | w |
| 1306 | w |
| 1295 | w |
| 1289 | w |
| 1273 | m |
| 1247 | m |
| 1227 | w |
| 1211 | m |
| 1177 | w |
| 1167 | w |
| 1122 | vw |
| 1106 | w |
| 1081 | w |
| 1076 | w |
| 1055 | m |
| 1047 | m |
| 1025 | w |
| 999 | vs |
| 971 | vw |
| 957 | m |
| 929 | w |
| 901 | w |
| 855 | m |
| 843 | m |
| 817 | w |
| 796 | w |
| 754 | m |
| 707 | vw |
| 676 | vw |
| 635 | m |
| 614 | w |
| 572 | vw |
| 536 | m |
| 514 | vw |
| 486 | vw |
| 468 | vw |
| 454 | w |
| 425 | vw |
| 400 | w |
| 359 | w |
| 345 | w |
| 328 | m |
| 292 | m |
| 272 | m |
| 247 | s |
| 188 | w |
| 118 | vs |
| 105 | vs |
| 81 | vs |

Ein weiterer Erfindungsgegenstand ist eine kristalline Form A von 3-[2-(Dimethylamino)methyl-(cyclohex-1-yl)]-phenol-maleat der Formel I umfassend eine oder mehrere der folgenden charakteristischen Banden im Raman Spektrum, jeweils ausgedrückt in Wellenzahlen (cm-1): 118 (vs), 188 (w), 400 (w), 676 (w), 2812 (w), 2879 (m),

vorzugsweise eine oder mehrere der folgenden Banden:
118 (vs), 188 (w), 292 (m), 328 (m), 359 (w), 400 (w), 486 (vw), 676 (w), 901 (w),1025 (w), 1273 (m), 1351 (m), 1412 (w), 1569 (vw), 1601 (m),1690 (m), 2812 (w), 2879 (m), 2986 (m), 3060 (m).

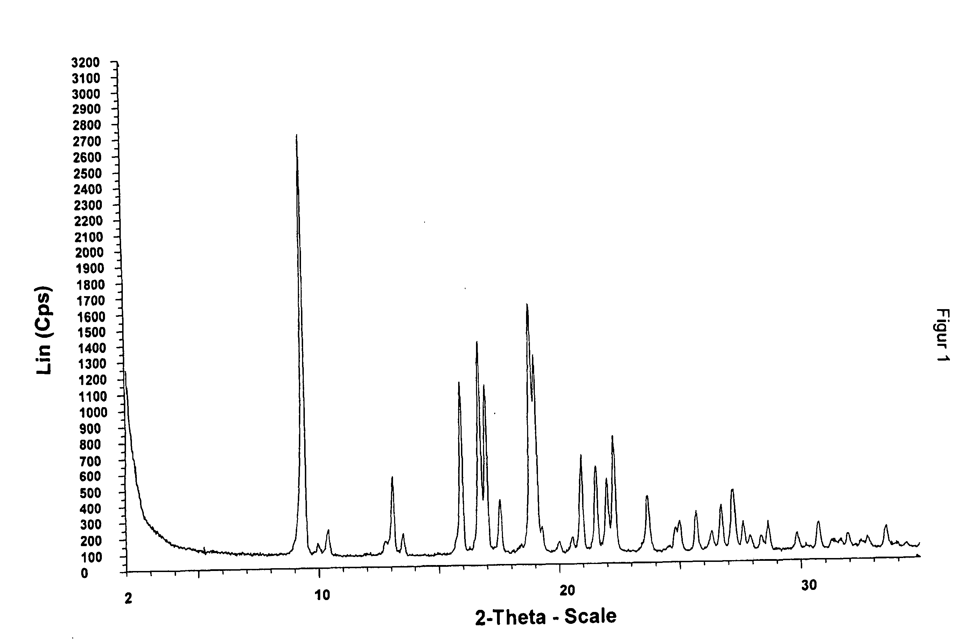

Ein weiterer Erfindungsgegenstand ist auch eine kristalline Form A von 3-[2-(Dimethylamino)methyl-(cyclohex-1-yl)]-phenol-maleat der Formel I, die ein Röntgenbeugungsbild wie in Figur 1 dargestellt aufweist.

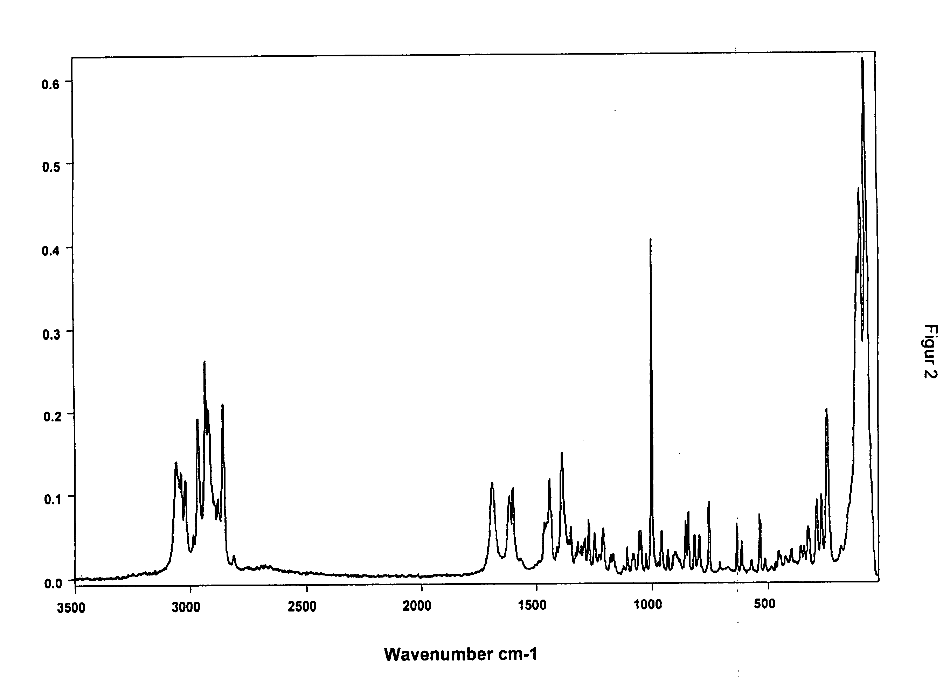

Ein weiterer Erfindungsgegenstand ist auch eine kristalline Form A von 3-[2-(Dimethylamino)methyl-(cyclohex-1-yl)]-phenol-maleat der Formel I, die ein Ramanspektrum wie in Figur 2 dargestellt aufweist.

Die kristalline Form A ist die thermodynamisch stabilste Form bei niedriger Temperatur bis zum Beispiel etwa 100 °C und weist zusätzlich eine ausgezeichnete chemische und physikalische Stabilität auf. Polymorph A ist sogar gegen den Einfluss von Luftfeuchtigkeit bei hohen relativen Luftfeuchtigkeiten bis zu 90% unempfindlich und stabil, selbst über einen längeren Zeitraum. Es wird keine Wasseraufnahme, keine Bildung von Hydraten und keine Umwandlung in andere kristalline Formen bei Normalbedingungen beobachtet. Polymorph A zeigt auch keine Phasentransformationen in Luft und Gegenwart von Feuchtigkeit. Polymorph A verändert sich auch nicht unter erhöhtem Druck oder beim Mahlen, und eine Umwandlung in andere kristalline Formen unter Einwirkung von höherem Druck findet nicht statt. Polymorph A ist auch nicht hygroskopisch und absorbiert nur geringe Mengen Oberflächenwasser. Polymorph A bildet unter diesen Bedingungen auch keine Solvate und im Kontakt mit Lösungsmitteln wird keine Umwandlung beobachtet. Die Löslichkeit in polaren Lösungsmitteln ist sehr gut. Der Schmelzpunkt beträgt etwa 167 °C und die Schmelzenthalpie beträgt etwa 135 J/g. Polymorph A kann als festes Pulver mit gewünschten mittleren Partikelgrössen hergestellt werden, die in der Regel im Bereich von 1 µm bis etwa 500 µm liegen. Die kristalline Form A eignet sich wegen dieser Eigenschaften hervorragend für die Herstellung pharmazeutischer Formulierungen.

Die Verbindung der Formel I bildet eine weitere, bei höheren Temperaturen thermodynamisch stabile kristalline Form B, die bei Normalbedingungen an Luft und unter Ausschluss von Luftfeuchtigkeit ebenfalls stabil ist. Die kristalline Form B ist auch so handhabbar, dass sie für die Herstellung pharmazeutischer Zusammensetzungen eingesetzt werden kann.

Ein weiterer Gegenstand der Erfindung ist eine kristalline Form von 3-[2-(Dimethylamino)methyl-(cyclohex-1-yl)]-phenol-maleat der Formel I, die ein charakteristisches Röntgen-Beugungsbild im Bereich von 2° bis 35° 2θ mit ausgeprägten charakteristischen Linien aufweist, ausgedrückt in d-Werten (Å): 10.6 (m), 7.5 (m), 7.3 (m), 6.1 (s), 5.29 (s) 4.88 (m), 4.72 (m), 4.47 (vs), 4.43 (m), 4.26 (m), 4.24 (m), 3.99 (s), 3.71 (m), 3.52 8m), 3.30 (s);
nachfolgend als Form B bezeichnet.

Ein weiterer Gegenstand der Erfindung ist eine kristalline Form B von 3-[2-(Dimethylamino)methyl-(cyclohex-1-yl)]-phenol-maleat der Formel I, die ein charakteristisches Röntgen-Beugungsbild mit den nachstehenden ausgeprägten Reflexen aufweist.

| **2Theta** | **rel. Intensität** |
|---|---|
| 8,36 | 18 |
| 11,85 | 20 |
| 12,19 | 20 |
| 13,41 | 12 |
| 14,50 | 49 |
| 14,83 | 11 |
| 16,77 | 58 |
| 18,16 | 17 |
| 18,78 | 28 |
| 19,84 | 100 |
| 20,00 | 17 |
| 20,84 | 19 |
| 20,97 | 16 |
| 21,19 | 12 |
| 22,27 | 33 |
| 22,64 | 7 |
| 22,85 | 11 |
| 23,95 | 18 |
| 24,53 | 7 |
| 24,85 | 8 |
| 25,26 | 16 |
| 25,97 | 8 |
| 26,66 | 12 |
| 27,00 | 29 |
| 27,18 | 13 |
| 27,48 | 8 |
| 28,21 | 4 |
| 29,10 | 6 |
| 29,41 | 9 |
| 29,90 | 8 |
| 30,49 | 10 |
| 31,32 | 6 |
| 33,14 | 6 |
| 33,88 | 5 |
| 34,32 | 4 |
| 34,99 | 10 |
| 36,32 | 3 |
| 36,88 | 4 |
| 37,86 | 4 |
| 38,35 | 10 |
| 42,11 | 5 |
| 42,78 | 3 |
| 43,05 | 3 |
| 43,62 | 6 |
| 44,35 | 4 |
| 46,83 | 6 |
| 47,80 | 3 |
| 48,35 | 3 |

In der vorstehenden Tabelle sind die Peaklagen (in 2 Theta) sowie die relativen Intensitäten der Peaks angegeben. Der intensivste Peak ist dabei auf eine relative Intensität von 100 normiert.

Ein weiterer Gegenstand der Erfindung ist Polymorph B des 3-[2-(Dimethylamino)methyl-(cyclohex-1-yl)]-phenol-maleats der Formel I, charakterisiert durch ein Pulverdiffraktogramm umfassend einen oder mehrere der folgenden Reflexe: 8,36 (18), 14,5 (49) und 14,83 (11) (jeweils ± 0,5 in 2 Theta, relative Intensität in Klammern). Vorzugsweise kann das Pulverdiagramm zusätzlich einen oder mehrere der folgenden Reflexe aufweisen: 11,85 (20), 12,19 (20), 18,16 (17), 22,85 (11), 29,1 (6) und 29,41 (6) (jeweils ± 0,2 in 2 Theta, relative Intensität in Klammern).

Ein anderer Gegenstand der Erfindung ist eine kristalline Form B von 3-[2-(Dimethylamino)methyl-(cyclohex-1-yl)]-phenol-maleat der Formel I, die ein charakteristisches Raman Spektrum mit charakteristischen Linien aufweist, ausgedrückt in Wellenzahlen (cm-1):

| Position (cm-1) | Intensität |
|---|---|
| 3045 | vs |
| 3028 | m |
| 2974 | s |
| 2953 | s |
| 2928 | vs |
| 2896 | s |
| 2856 | s |
| 2829 | m |
| 1703 | s |
| 1621 | m |
| 1612 | m |
| 1583 | m |
| 1480 | vw |
| 1459 | m |
| 1446 | m |
| 1401 | vw |
| 1385 | m |
| 1360 | s |
| 1227 | vw |
| 1324 | w |
| 1313 | m |
| 1309 | m |
| 1295 | m |
| 1282 | m |
| 1253 | w |
| 1220 | w |
| 1211 | m |
| 1197 | m |
| 1178 | w |
| 1162 | m |
| 1124 | w |
| 1104 | w |
| 1081 | w |
| 1076 | w |
| 1052 | m |
| 1010 | w |
| 999 | vs |
| 971 | vw |
| 956 | w |
| 933 | w |
| 890 | w |
| 875 | m |
| 858 | m |
| 841 | s |
| 816 | m |
| 794 | m |
| 750 | m |
| 708 | vw |
| 628 | w |
| 620 | w |
| 605 | vw |
| 578 | w |
| 573 | w |
| 538 | m |
| 507 | vw |
| 472 | w |
| 455 | w |
| 449 | w |
| 418 | w |
| 372 | w |
| 340 | m |
| 307 | w |
| 272 | m |
| 246 | s |
| 229 | m |
| 101 | vs |
| 83 | vs |

Ein weiterer Erfindungsgegenstand ist eine kristalline Form B von 3-[2-(Dimethylamino)methyl-(cyclohex-1-yl)]-phenol-maleat der Formel I umfassend eine oder mehrere der folgenden charakteristischen Banden im Raman Spektrum, jeweils ausgedrückt in Wellenzahlen (cm-1): 229 (m), 875 (m) und 2829 (m),

vorzugsweise eine oder mehrere der folgenden Banden:
229 (m), 307 (w), 372 (w), 605 (vw), 875 (m), 890 (w), 1010 (w), 1197 (m), 1401 (vw), 1480 (vw), 1583 (m), 1703 (s), 2829 (m), 2953 (s).

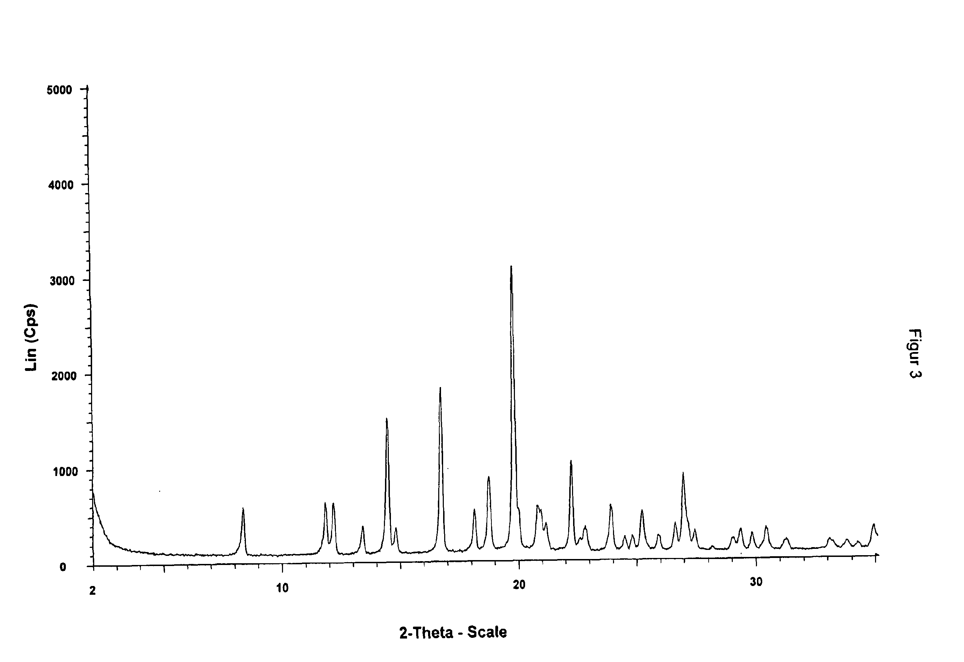

Erfindungsgegenstand ist auch eine kristalline Form B von 3-[2-(Dimethylamino)methyl-(cyclohex-1-yl)]-phenol-maleat der Formel I, die ein Röntgenbeugungsbild wie in Figur 3 dargestellt aufweist.

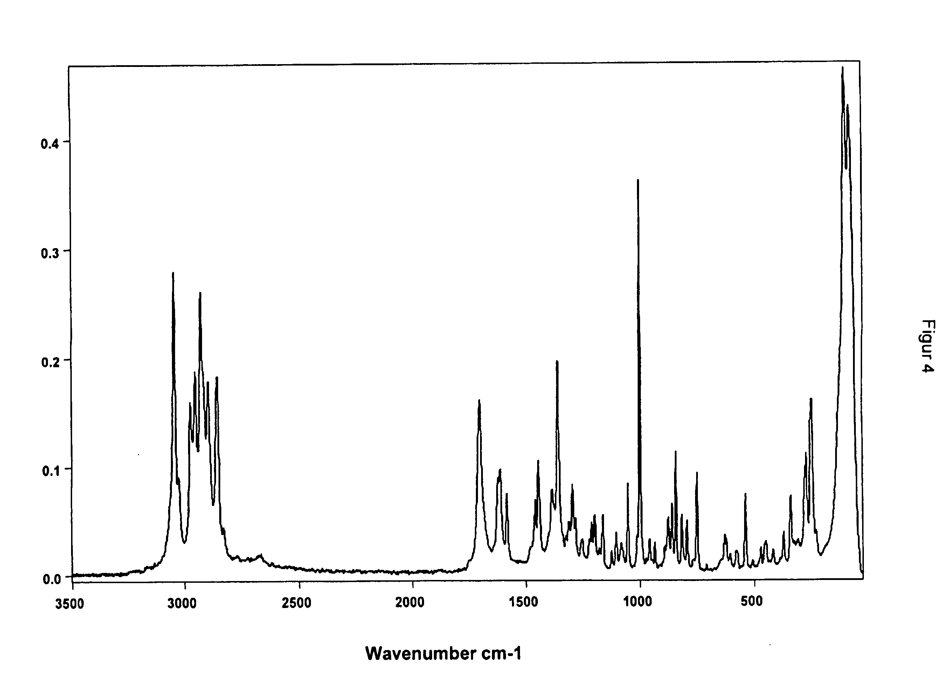

Erfindungsgegenstand ist weiter eine kristalline Form B von 3-[2-(Dimethylamino)methyl-(cyclohex-1-yl)]-phenol-maleat der Formel I, die ein Ramanspektrum wie in Figur 4 dargestellt aufweist.

Die polymorphe Form B kann sich in die kristalline Form A umwandeln. Die Formen A und B bilden ein enantiotropes System mit einem Übergangspunkt bzw. -bereich von etwa 100 bis 120 °C. Gegenstand der Erfindung sind daher auch Mischungen der kristallinen Formen A und B in an sich beliebigen Mengenverhältnissen.

Die kristalline Form B ist die thermodynamisch stabilste Form bei höherer Temperatur, zum Beispiel etwa mindestens 100 °C oder mehr. Polymorph B weist zusätzlich eine ausgezeichnete chemische Stabilität bei erhöhten Temperaturen auch in Gegenwart von Luftfeuchtigkeit auf. Die physikalische Stabilität ist geringer, denn bei erhöhtem Druck, und auch höheren Temperaturen mit oder ohne Feuchtigkeitseinfluss tritt eine Umwandlung in Polymorph A ein. Es wird aber keine Wasseraufnahme und keine Bildung von Hydraten beobachtet. Polymorph B bildet auch keine Solvate. Im Kontakt mit Lösungsmitteln kann eine Umwandlung in die kristalline Form A eintreten. Die Löslichkeit in polaren Lösungsmitteln ist sehr gut und vergleichbar mit der Löslichkeit von Polymorph A. Der Schmelzpunkt beträgt etwa 178 °C und die Schmelzenthalpie beträgt etwa 125 J/g. Polymorph B kann als festes Pulver mit gewünschten mittleren Partikelgrössen hergestellt werden, die in der Regel im Bereich von 1 µm bis etwa 500 µm liegen. Eine Lagerung der kristallinen Form B unter trockenem Schutzgas (Stickstoff) ist empfehlenswert.

Die polymorphen Formen können nach an sich bekannten Kristallisationsverfahren aus dem Salz 3-[2-(Dimethylamino)methyl-(cyclohex-1-yl)]-phenol-maleat hergestellt werden, zum Beispiel Rühren von Suspensionen (Einstellung von Phasengleichgewichten), Fällung, Umkristallisation, oder Verdampfung von Lösungsmitteln. Es können verdünnte, gesättigte oder übersättigte Lösungen verwendet werden, mit oder ohne Animpfen mit einem Kristallkeimbildner. Die Temperaturen zur Bildung von Lösungen können bis zu 100 °C betragen. Die Kristallisation kann durch Kühlen auf etwa -100 °C bis 30 °C, und bevorzugt -30 °C bis 20 °C initiiert werden, wobei ein Abkühlen kontinuierlich oder stufenweise erfolgen kann. Zur Herstellung von Lösungen oder Suspensionen können amorphe oder kristalline Startmaterialien verwender werden, um hohe Konzentrationen in Lösungen zu erzielen und andere kristalline Formen zu erhalten.

Gegenstand der Erfindung ist auch ein Verfahren zur Herstellung der kristallinen Form A von 3-[2-(Dimethylamino)methyl-(cyclohex-1-yl)]-phenol-maleat, das dadurch gekennzeichnet ist, dass man
a) eine pulverförmige, feste, amorphe Form von 3-[2-(Dimethylamino)methyl-(cyclohex-1-yl)]-phenol-maleat mit einem Wasserdampf enthaltenden inerten Gas behandelt; oder
b) eine Suspension der amorphen Form von 3-[2-(Dimethylamino)methyl-(cyclohex-1-yl)]-phenol-maleat in einem Lösungsmittel als Träger herstellt und bis zur vollständigen Bildung der kristallinen Form A rührt; oder
c) 3-[2-(Dimethylamino)methyl-(cyclohex-1-yl)]-phenol-maleat in einem Lösungsmittel löst und anschliessend ausfällt; oder
d) 3-[2-(Dimethylamino)methyl-(cyclohex-1-yl)]-phenol und Maleinsäure vereinigt,
wobei vorzugsweise wenigstens eine dieser Komponenten in gelöster oder suspendierter Form vorliegt, und das erhaltene Produkt isoliert, mit der Maßgabe, dass die Temperatur nicht über 100 °C beträgt.

Die Temperatur in den Verfahrensschritten a) und b) beträgt bevorzugt höchstens 40 °C und das Verfahren wird besonders bevorzugt bei Raumtemperatur durchgeführt. Inerte Gase sind zum Beispiel Luft, Stickstoff und Edelgase, wobei Luft schon aus wirtschaftlichen Gründen besonders bevorzugt ist. Die relative Feuchtigkeit der Gase kann zum Beispiel 40 bis 90% und bevorzugt 60 bis 90 % betragen. Die Behandlungszeit in Verfahrensstufe a) hängt im wesentlichen von der Partikelgrösse und der relativen Feuchtigkeit ab und kann zum Beispiel 5 bis 100 Stunden betragen.

In Verfahrensstufe a) kann auch das Polymorph B neben der Form A gebildet werden, wenn die relative Luftfeuchtigkeit zu niedrig und/oder die Behandlungszeit zu kurz ist. Nach der Isolierung kann der kristalline Rückstand in üblicher Weise getrocknet werden, wobei man zweckmässig Temperaturen über 100 °C vermeidet.

Der Verfahrensschritt b) wird vorzugsweise bei etwa -20 ° bis 40 °C und besonders bevorzugt bei -5 °C bis 25 °C (etwa Raumtemperatur) durchgeführt. In Frage kommende Lösungsmittel sind zuvor genannt worden. Die Behandlungszeit in Verfahrensstufe b) kann 5 bis 100 Stunden betragen. Nach der Isolierung kann das verwendete Lösungsmittel beziehungsweise Lösungsmittelgemisch in üblicher Weise mittels bekannter Trocknungsverfahren entfernt werden.

Im Verfahrensschritt c) kann die kristalline Form A, B oder die amorphe Form von 3-[2-(Dimethylamino)methyl-(cyclohex-1-yl)]-phenol-maleat zur Herstellung von Lösungen verwendet werden. Die Konzentration an 3-[2-(Dimethylamino)methyl-(cyclohex-1-yl)]-phenol-maleat in der Lösung hängt von der gewählten Temperatur und vom Lösungsmittel ab. Die gelöste Menge kann zum Beispiel von 0,5 bis 50, bevorzugt 0,5 bis 30, bevorzugter 0,5 bis 20 und besonders bevorzugt 1 bis 15 Gewichtsprozent betragen, bezogen auf das Lösungsmittel. Die Temperatur zum Lösen kann bis zu 100 °C und bevorzugt bis zu 60 °C betragen. Die Lösung kann auch bei Raumtemperatur hergestellt werden, wenn man Lösungsmittel mit hohem Lösungsvermögen einsetzt, zum Beispiel Wasser, Aceton, Dimethylformamid, Dimethylsulfoxid, Methanol, Ethanol, N-Methylpyrrolidon und Propylenglykol. Die Fällung kann mittels Abkühlen, teilweiser oder vollständiger Entfernung des Lösungsmittels, Zugabe eines Fällungsmittels (Nichtlösungsmittel, zum Beispiel Heptan oder Methylt-butylether) oder einer Kombination dieser Massnahmen erfolgen. Abkühlen kann langsames Abkühlen oder auch Abschrecken auf Temperaturen bis -20 °C und bevorzugt bis 0°C bedeuten. Das Lösungsmittel kann durch Erwärmen, im Gasstrom, Anlegen von Vakuum oder einer Kombination dieser Massnahmen erfolgen. Erwärmen zum Entfernen von Lösungsmittel bedeutet in der Verfahrensstufe c) eine Temperatur von höchstens 70 und vorzugsweise höchstens 50 °C. Fällungsverfahren gemäss Verfahrensschritt c) werden bevorzugt für die Herstellung der polymorphen Form A eingesetzt.

Im Verfahrenschritt d) beträgt die Temperatur bevorzugt nicht mehr als 80 °C, bevorzugter nicht mehr als 70 °C und besonders bevorzugt nicht mehr als 60 °C. Die Temperatur liegt beim Lösen bzw. Suspendieren einer Komponente im allgemeinen höher als beim Vermischen.

Ein weiterer Gegenstand der vorliegenden Erfindung ist ein Salz der Formel I von Maleinsäure und 3-[2-(Dimethylamino)methyl-(cyclohex-1-yl)]-phenol in der kristallinen Form A (Polymorph A) erhältlich nach einem der vorstehend beschriebenen Verfahren.

Während die Herstellung der polymorphen Form A relativ unkritisch ist, kann die polymorphe Form B nur mit einem speziellen Verfahren erhalten werden. Es wurde überraschend gefunden, dass Form B mit einem Verdampfungsverfahren, einer spezifischen Kombination von Lösungsmittel und hohen Verdampfungstemperaturen reproduzierbar zugänglich ist.

Ein anderer Gegenstand der Erfindung ist daher ein Verfahren zur Herstellung der kristallinen Form B von 3-[2-(Dimethylamino)methyl-(cyclohex-1-yl)]-phenol-maleat, das dadurch gekennzeichnet ist, dass man 3-[2-(Dimethylamino)methyl-(cyclohex-1-yl)]-phenol-maleat in einer Mischung aus Tetrahydrofuran und Wasser im Volumenverhältnis von 0,8:1,2 bis 1,2:0,8 löst, und dann das Lösungsmittelgemisch bei einer Temperatur von mindestens 80 °C vollständig entfernt. Es sind auch tiefere Verdampfungstemperaturen in offener Atmosphäre möglich, zum Beispiel bei Raumtemperatur in einem offenen Gefäss (Kontakt mit Luftfeuchtigkeit).

Tetrahydrofuran (THF) und Wasser weisen vorzugsweise ein Volumenverhältnis von 1:1 auf. Vor der Entfernung des Lösungsmittelgemisches kann die Lösung für einen bestimmten Zeitraum, zum Beispiel bis zu 24 Stunden bei erhöhter Temperatur (etwa 80 bis 100 °C) gerührt werden. Zur Entfernung des Lösungsmittelgemisches kann zusätzlich ein Gasstrom angewendet und/oder ein Vakuum angelegt werden. Erwärmen zum Entfernen von Lösungsmittel bedeutet eine Temperatur von mindestens 80 und vorzugsweise bis etwa 120 °C und besonders bevorzugt 90 bis 100 °C. Das Lösungsmittel wird besonders bevorzugt am Rotationsverdampfer abdestilliert. Die Menge an 3-[2-(Dimethylamino)methyl-(cyclohex-1-yl)]-phenol-maleat im Gemisch aus THF und Wasser kann insbesondere 0,5 bis 20, bevorzugt 0,5 bis 10 Gewichtsprozent betragen, bezogen auf das Gemisch THF und Wasser.

Es wurde auch gefunden, dass Einstellungen von Phasengleichgewichten mittels Rühren von Suspensionen bei Temperaturen von mindestens 120 °C zur Bildung der kristallinen Form B führen.

Ein weiterer Gegenstand der vorliegenden Erfindung ist ein Salz der Formel I von Maleinsäure und 3-[2-(Dimethylamino)methyl-(cyclohex-1-yl)]-phenol in der kristallinen Form B (Polymorph B) erhältlich nach einem der vorstehend beschriebenen Verfahren.

3-[2-(Dimethylamino)methyl-(cyclohex-1-yl)]-phenol-maleat ist auf Grund seines günstigen Gesamteigenschaftsprofils hervorragend als Wirkstoff für pharmazeutische Zusammensetzungen und ganz besonders für schmerzstillende Medikamente geeignet. Demgemäss ist auch ein Erfindungsgegenstand die Verwendung von 3-[2-(Dimethylamino)methyl-(cyclohex-1-yl)]-phenol-maleat der Formel I als Wirkstoff in Arzneimitteln, bevorzugt als Wirkstoff in Schmerzmitteln. Bevorzugt sind auch hier, wie in der ganzen Anmeldung, Diastereomere oder Gemische von enantiomeren Diastereomeren mit Transkonfiguration des Phenylringes und der Dimethylaminomethylgruppe (1 R,2R- beziehungsweise 1S,2S-Konfiguration), wobei das Enantiomer mit der absoluten Konfiguration (1 R,2R) ganz besonders bevorzugt ist.
Ein weiterer Gegenstand der Erfindung ist eine pharmazeutische Zusammensetzung, enthaltend eine wirksame Menge 3-[2-(Dimethylamino)methyl-(cyclohex-1-yl)]-phenol-maleat der Formel I und einen pharmazeutischen Träger oder ein pharmazeutisches Verdünnungsmittel.

In der Zusammensetzung kann die Verbindung der Formel I als kristalline Form A, kristalline Form B oder einer Mischung der Formen A und B vorliegen. Vorzugsweise ist die kristalline Form A enthalten.

Die Menge an Verbindungen der Formel I hängt im wesentlichen vom Formulierungstyp und von der gewünschten Dosierung während des Zeitraums der Verabreichung ab. Die an den Patienten zu verabreichende Menge der jeweiligen erfindungsgemäßen Salze kann variieren und ist beispielsweise abhängig vom Gewicht oder Alter des Patienten sowie von der Applikationsart, der Indikation und dem Schweregrad der Erkrankung. Üblicherweise werden 0,005 bis 5000 mg/kg, vorzugsweise 0,05 bis 500 mg/kg Körpergewicht des Patienten wenigstens einer solchen Verbindung appliziert.

Bei oralen Formulierungen kann es sich um feste Formulierungen handeln, zum Beispiel Tabletten, Kapseln, Pillen und Pastillen. Bei oralen Formulierungen kann es sich auch um flüssige Formulierungen handeln, zum .Beispiel Lösungen, Suspensionen, Sirupe oder Elexire. Flüssige und feste Formulierungen umfassen auch die Einarbeitung der Verbindungen der Formel I in feste oder flüssige Nahrungsmittel. Ferner umfassen Flüssigkeiten noch Lösungen für parenterale Applikationen wie zum Beispiel Infusion oder Injektion.

Die Verbindungen der Formel I und die kristallinen Formen können direkt als Pulver (mikronisierte Teilchen), Granulate, Suspensionen oder Lösungen verwendet werden, oder sie können mit anderen pharmazeutisch annehmbaren Zutaten und Komponenten vermischt und dann pulverisiert werden, um die Pulver dann in Kapseln aus Hart- oder Weichgelatine zu füllen, Tabletten, Pillen oder Pastillen zu pressen, oder um die Pulver in einem Träger zu suspendieren oder zu lösen zur Herstellung von Suspensionen, Sirupen oder Elexieren. Tabletten, Pillen oder Pastillen können nach dem Pressen mit einem Überzug versehen werden.

Pharmazeutisch annehmbare Zutaten und Komponenten für die verschiedenen Formulierungstypen sind an sich bekannt. Es kann sich zum Beispiel um Bindemittel wie synthetische oder natürliche Polymere, Arzneiträger, Gleitmittel, Tenside, Süssmittel und Aromastoffe, Beschichtungsmittel, Konservierungsmittel, Farbstoffe, Verdickungsmittel, Hilfsmittel, antimikrobielle Mittel und Träger für die verschiedenen Formulierungstypen handeln.

Beispiele für Bindemittel sind Gummi Arabicum, Gummi Tragakant, Akaziengummi und biologisch abbaubare Polymere wie Homo- oder Copolyester von Dicarbonsäuren, Alkylendiolen, Polyalkylenglykolen und/oder aliphatischen Hydroxycarbonsäuren; Homo- oder Copolyamide von Dicarbonsäuren, Alkylendiaminen und/oder aliphatischen Aminocarbonsäuren; entsprechende Polyester-polyamid-copolymere, Polyanhydride, Polyorthoester, Polyphosphazene und Polycarbonate. Die biologisch abbaubaren Polymere können linear, verzeigt oder vernetzt sein. Spezifische Beispiele sind Polyglykolsäure, Polymilchsäure und Poly-d,1-milch-/ glykolsäure. Andere Beispiele für Polymere sind wasserlösliche Polymere wie zum Beispiel Polyoxaalkylene (Polyoxaethylen, Polyoxapropylen und Mischpolymere davon), Polyacrylamide und hydroxylalkylierte Polyacrylamide, Polymaleinsäure und Ester oder Amide davon, Polyacrylsäure und Ester oder Amide davon, Polyvinylalkohol und Ester oder Ether davon, Polyvinylimidazol, Polyvinylpyrrolidon und natürliche Polymere wie zum Beispiel Chitosan.

Beispiele für Arzneiträger sind Phosphate wie Dicalciumphosphat.

Beispiele für Gleitmittel sind natürliche oder synthetische Öle, Fette, Wachse oder Fettsäuresalze wie Magnesiumstearat.

Tenside (oberflächenaktive Mittel) können anionisch, kationisch, amphoter oder neutral sein. Beispiele für Tenside sind Lecithin, Phospholipide, Octylsulfat, Decylsulfat, Dodecylsulfat, Tetradecylsulfat, Hexadecylsulfat und Octadecylsulfat, Natriumoleat oder Natriumcaprat, 1-Acylaminoethan-2-sulfonsäuren wie 1-Octanoylaminoethan-2-sulfonsäure, 1-Decanoylaminoethan-2-sulfonsäure, 1-Dodecanoylaminoethan-2-sulfonsäure, 1-Tetradecanoylaminoethan-2-sulfonsäure, 1-Hexadecanoylaminoethan-2-sulfonsäure und 1-Octadecanoylaminoethan-2-sulfonsäure, Gallensäuren, Salze und Derivate davon, wie zum Beispiel Cholsäure, Deoxycholsäure, Taurocholsäure, Taurodeoxycholsäure und Natriumglycocholate, Natriumcaprat, Natriumlaurat, Natriumoleat, Natriumlaurylsulfat, Natriumcetylsulfat, sulfatiertes Castoröl, Natriumdioctylsulfosuccinat, Cocamidopropylbetain und Laurylbetain, Fettalkohole, Cholesterole, Glycerinmono- oder -distearat, Glycerinmono- oder -dioleat, Glycerinmono- oder -dipalmitat und Polyoxyethylenstearat.

Beispiele für Süssmittel sind Sucrose, Fructose, Lactose oder Aspartam.

Beispiele für Aromastoffe sind Pfefferminz, Öl von Wintergrün oder Fruchtaroma wie Kirschen- oder Orangenaroma.

Beispiele für Beschichtungsmittel sind Gelatine, Wachse, Schellack, Zucker oder biologisch abbaubare Polymere.

Beispiele für Konservierungsmittel sind Methyl- oder Propylparaben, Sorbinsäure, Chlorbutanol und Phenol.

Beispiele für Hilfsmittel sind Duftstoffe.

Beispiele für Verdickungsmittel sind synthetische Polymere, Fettsäuren, Fettsäuresalze, Fettsäureester und Fettalkohole.

Beispiele für flüssige Träger sind Wasser, Alkohole (Ethanol, Glycerol, Propylenglykol, flüssige Polyethylenglykole, Polytriazine und Öle. Beispiele für feste Träger sind Talk, Tonerden, mikrokristalline Cellulose, Siliziumdioxid, Aluminiumoxid und ähnliche Feststoffe.

Die erfindungsgemässe Zusammensetzung kann auch isotonische Mittel wie zum Beispiel Zucker, physiologische Puffer und Natriumchlorid enthalten.

Die erfindungsgemässe Zusammensetzung kann auch als Brausetablette oder Brausepulver formuliert sein, die sich in wässriger Umgebung zersetzt unter Zubereitung von Trinklösungen oder -suspensionen.

Ein Sirup oder Elexier kann die Verbindung der Formel I, einen Zucker wie Sucrose oder Fructose als Süssmittel, ein Konservierungsmittel (wie Methylparaben), einen Farbstoff und ein Geschmacksmittel (wie Aromastoffe) enthalten.

Bei der erfindungsgemässen Zusammensetzung kann es sich auch um Formulierungen mit verzögerter und kontrollierter Wirkstoffabgabe im Kontakt mit Körperflüssigkeiten des gastrointestinalen Traktes handeln, um einen im wesentlichen konstanten und effektiven Level des Wirkstoffs im Blutplasma zu erzielen. Die Verbindungen der Formel I können für diesen Zweck in eine Polymermatrix eines biologisch abbaubaren Polymers, eines wasserlöslichen Polymers oder beiden Polymeren eingebettet werden, gegebenenfalls zusammen mit einem geeigneten Tensid. Einbetten kann in diesem Zusammenhang die Einarbeitung von Mikropartikeln in die Polymermatrix bedeuten. Formulierungen mit verzögerter und kontrollierter Wirkstoffabgabe können auch mittels Enkapsulierung von dispergierten Mikropartikeln oder emulgierten Mikrotropfen über bekannte Beschichtungstechnologien von Dispersionen und Emulsionen erhalten werden.

Die Verbindungen der Formel I können auch zusammen mit wenigstens einem weiteren pharmazeutischen Wirkstoff für Kombinationstherapien verwendet werden. Hierzu kann wenigstens ein weiterer Wirkstoff zusätzlich in der erfindungsgemässen Zusammensetzung dispergiert oder gelöst werden.

Gegenstand der Erfindung ist auch die Verwendung von 3-[2-(Dimethylamino)methyl-(cyclohex-1-yl)]-phenol-maleat der Formel I zur Herstellung einer pharmazeutischen Zusammensetzung, insbesondere für die Behandlung von Schmerzzuständen.

Ein weiterer Gegenstand der Erfindung ist ein Verfahren zur Behandlung von Schmerzzuständen, bei dem man einem unter Schmerzen leidenden Patienten eine wirksame Menge von 3-[2-(Dimethylamino)methyl-(cyclohex-1-yl)]-phenol-maleat der Formel I verabreicht.

Bevorzugt eignet sich das erfindungsgemäße Arzneimittel (die erfindungsgemäße pharmazeutische Zusammensetzung) zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz; von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS), Panikattacken; Epilepsie; Husten; Harninkontinenz; Diarrhöe; Pruritus; Schizophrenie; cerebralen Ischämien; Muskelspasmen; Krämpfen; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch; Alkohol- und/oder Drogen (insbesondere Nikotin- und/oder Kokain-) und/oder Medikamentenabhängigkeit, vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; Entwicklung von Toleranzerscheinungen gegenüber Medikamenten; insbesondere gegenüber Opioiden; des Magen-Ösaphagus-Reflux-Syndroms; zur Diurese; zur Antinatriurese; zur Beeinflussung des kardiovaskulären Systems; zur Anxiolyse; zur Vigilanzsteigerung; zur Libidosteigerung, zur Modulation der Bewegungsaktivität und zur Lokalanästhesie.

Besonders bevorzugt eignet sich das erfindungsgemäße Arzneimittel (die erfindungsgemäße pharmazeutische Zusammensetzung) zur Prophylaxe und/oder Behandlung von Schmerzen, vorzugsweise von akuten Schmerzen, chronischen Schmerzen, neuropathischen Schmerzen oder visceralen Schmerzen; Depressionen; Epilepsie; Morbus Parkinson; Alkohol- und/oder Drogen (insbesondere Nikotin- und/oder Kokain-) und/oder Medikamentenmißbrauch; Alkohol- und/oder Drogen (insbesondere Nikotin- und/oder Kokain-) und/oder Medikamentenabhängigkeit; vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; der Entwicklung von Toleranzerscheinungen gegenüber Medikamenten, insbesondere gegenüber Opioiden, oder zur Anxiolyse.

Ganz besonders bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen.

Besonders bevorzugt ist die Verwendung wenigstens eines erfindungsgemäßen Salzes , jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, sowie ggf. eines oder mehrerer pharmazeutisch verträglicher Hilfsstoffe zur Herstellung eines Arzneimittels zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz, von Migräne, Depressionen, neurodegenerativen Erkrankungen, vorzugsweise ausgewählt aus der Gruppe bestehend aus Morbus Parkinson, Morbus Alzheimer, Morbus Huntington und Multipler Sklerose, kognitiven Erkrankungen, vorzugsweise kognitiven Mangelzuständen, besonders bevorzugt des Aufmerksamkeit-Defizit-Syndroms (ADS), Panikattacken, Epilepsie, Husten, Harninkontinenz, Diarrhöe, Pruritus, Schizophrenie, cerebralen Ischämien, Muskelspasmen, Krämpfen, Störungen der Nahrungsaufnahme, vorzugsweise ausgewählt aus der Gruppe bestehend aus Bulimie; Kachexie, Anorexie und Fettleibigkeit, Alkohol- und/oder Drogen (insbesondere Nikotin- und/oder Kokain-) und/oder Medikamentenmißbrauch; Alkohol- und/oder Drogen (insbesondere Nikotin- und/oder Kokain-) und/oder Medikamentenabhängigkeit;vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; Entwicklung von Toleranzerscheinungen gegenüber Drogen- und/oder Medikamenten, insbesondere gegenüber Opioiden, des Magen-Ösaphagus-Reflux-Syndroms, zur Diurese, zur Antinatriurese, zur Beeinflussung des kardiovaskulären Systems, zur Anxiolyse, zur Vigilanzsteigerung, zur Libidosteigerung, zur Modulation der Bewegungsaktivität und zur Lokalanästhesie.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem erfindungsgemäßen Salz, ggf. in Form seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, , enthält das erfindungsgemäße Arzneimittel (die erfindungsgemäße pharmazeutische Zusammenstzung) üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel (die pharmazeutische Zusammensetzung) oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Geeignete perkutane Applikationszubereitungen sind auch Depotzubereitungen in gelöster Form oder in einem Pflaster, gegebenenfalls unter Zusatz von die Hautpenetration fördernden Mitteln.

Oral oder perkutan anwendbare Zubereitungsformen können die jeweiligen erfindungsgemäßen Salze verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind Die entsprechende Beschreibung wird hiermit als Referenz eingeführt und gilt als Teil der Offenbarung.

Die an den Patienten zu verabreichende Menge der jeweiligen erfindungsgemäßen Salze kann variieren und ist beispielsweise abhängig vom Gewicht oder Alter des Patienten sowie von der Applikationsart, der Indikation und dem Schweregrad der Erkrankung. Üblicherweise werden 0,005 bis 5000 mg/kg, vorzugsweise 0,05 bis 500 mg/kg Körpergewicht des Patienten wenigstens einer solchen Verbindung appliziert.

Die nachfolgenden Beispiele erläutern die Erfindung näher, ohne sie hierauf zu beschränken.

### Beispiele:

Bei allen DSC Messungen (wenn nicht anders angegeben) betragen die Aufheizraten 10°C / Minute; die angebenen Temperaturen sind Peakmaxima.

### A) Herstellung von (+)-(1R,2R)-3-[2-(Dimethylamino)methyl-(cyclohex-1-yl)]-phenol-maleat

### Beispiel A1: Herstellung als kristalline Form A

0,0685 g (+)-(1 R,2R)-3-[2-(Dimethylamino)methyl-(cyclohex-1-yl)]-phenol werden in 0,4 ml Ethylacetat gelöst. Daneben wird eine zweite Lösung von 0,0371 g Maleinsäure in 1 ml Ethylacetat zubereitet. Die beiden Lösungen werden bei Raumtemperatur zusammengegeben und unter Rühren vermischt, wobei sich sofort ein weisser Niederschlag bildet. Die Mischung wird unter Rühren auf 50 °C erwärmt und danach wieder auf Raumtemperatur abgekühlt. Dann filtriert man den Niederschlag über eine Glasfritte ab, wäscht mit 4 ml Ethylacetat und trocknet den Rückstand, indem man während 5 Minuten Luft durchsaugt. Man erhält 0,0870 mg (85% der Theorie) (+)-(1R,2R)-3-[2-(Dimethylamino)methyl-(cyclohex-1-yl)]-phenol-maleat als weissen, kristallinen Feststoff mit einem Schmelzpunkt von etwa 167 °C und die Schmelzenthalpie beträgt etwa 135 J/g, bestimmt mittels Differential-Scanning Kalorimetrie (DSC) bei einer Aufheizrate von 10 °C/Minute. Der kristalline Feststoff ist die polymorphe Form A, deren Röntgen-Beugungsbild in Figur 1 dargestellt ist. Das Raman-Spektrum ist in Figur 2 dargestellt.

Wenn man die doppelte Menge an (+)-(1 R,2R)-3-[2-(Dimethylamino)methyl-(cyclohex-1-yl)]-phenol einsetzt, wird ebenfalls nur das 1:1-Maleat gebildet.

### Beispiel A2: Herstellung als kristalline Form A

6,22 g (53,5 mmol) Maleinsäure werden in einem Glaskolben in 130 ml Essigsäureethylester gelöst und dann unter Rühren zu einer auf 50 °C temperierten Lösung von 12,48 g (53,5 mmol) (+)-(1 R,2R)-3-[2-(Dimethylamino)methyl-(cyclohex-1-yl)]-phenol in 40 ml Essigsäureethylester getropft. Nach Zugabe von 20 bis 25 ml bildet sich ein weisser Niederschlag. Nach Zugabe der gesamten Lösung wird der feinkristalline, weisse Feststoff aus der warmen Lösung abfiltriert und mit wenig kaltem Essigsäureethylester gewaschen. Der Rückstand wird mittels Durchsaugen von Luft getrocknet. Man erhält 18,38 g (98,3% der Theorie) (+)-(1 R,2R)-3-[2-(Dimethylamino)methyl-(cyclohex-1-yl)]-phenol-maleat als kristalline Form A (gemäss Röntgen-Beugungsbild) mit einem Festpunkt von 177-179 °C. Mittels DSC Analyse werden zwei Peaks bei 165,5 und 177,2 °C gefunden.
10 g des Feststoffs werden im Trockenschrank bei 40 °C und 80 bis 90 mbar über Nacht getrocknet. Der Gewichtsverlust beträgt nur 3,8 mg (Ethylacetat). Das Röntgen-Beugungsbild entspricht der Form A; die DSC Peaks sind fast unverändert 165,8 und 176,7.

### Beispiel A3: Herstellung als kristalline Form B

In einem Glaskolben werden 1,95 g (+)-(1 R,2R)-3-[2-(Dimethylamino)methyl-(cyclohex-1-yl)]-phenol bei Raumtemperatur in 40 ml Essigsäureethylester gelöst. Dann wird bei Raumtemperatur eine Lösung von 0,97 g Maleinsäure in 25 ml Essigsäureethylester zugetropft. Nach Zugabe von etwa der Hälfte der Lösung bildet sich erst eine Trübung und dann ein kristalliner Niederschlag. Die Suspension wird bei 70 °C eine Stunde am Rotationsverdampfer gerührt und dann auf Raumtemperatur abgekühlt. Die Suspension wird dann im Kühlschrank auf 4 °C gekühlt und der Niederschlag abfiltriert. Der feste Rückstand wird einmal mit 4 ml Essigsäureethylester gewaschen und mittels Durchsaugen von Luft (5 Minuten) getrocknet. Man erhält 2,815 g (96,4% der Theorie) (+)-(1 R,2R)-3-[2-(Dimethylamino)methyl-(cyclohex-1-yl)]-phenol-maleat als kristalline Form B (gemäss Röntgen-Beugungsbild) mit einem Festpunkt von 177-179 °C. Mittels DSC Analyse wird ein Peak bei 177,7 °C gefunden.

### Beispiel A4: Herstellung als kristalline Form A

8,92 g Maleinsäure werden bei 55 °C in einem Glaskolben in 120 ml Essigsäureethylester gelöst. In einem anderen Glaskolben In einem Glaskolben werden ebenfalls bei 55 °C 17,96 g (+)-(1 R,2R)-3-[2-(Dimethylamino)methyl-(cyclohex-1-yl)]-phenol bei Raumtemperatur in 108 ml Essigsäureethylester gelöst und langsam innerhalb von 30 Minuten zu der Lösung von Maleinsäure getropft. Nach Zugabe von etwa der Hälfte fällt ein weisser, voluminöser feinkristalliner Feststoff aus. Die Suspension wird nach vollständiger Zugabe noch 3 Stunden bei 55 °C gerührt. Anschliessend lässt man von selbst auf Raumtemperatur abkühlen und rührt noch über Nacht. Der Niederschlag wird abfiltriert mit 20 ml Essigsäureethylester gewaschen und mittels Durchsaugen von Luft (5 Minuten) getrocknet. Man erhält 26,26 g (97,7% der Theorie) (+)-(1 R,2R)-3-[2-(Dimethylamino)methyl-(cyclohex-1-yl)]-phenol-maleat als kristalline Form A (gemäss Röntgen-Beugungsbild). Mittels DSC Analyse werden zwei Peaks bei 166,03 und 177,03 °C gefunden.

### Beispiel A5: Herstellung als kristalline Form A

In je einem Glaskolben werden 2,33 g (+)-(1 R,2R)-3-[2-(Dimethylamino)methyl-(cyclohex-1-yl)]-phenol bei Raumtemperatur in 15 ml Essigsäureethylester gelöst beziehungsweise 1,16 g Maleinsäure in 30 ml Essigsäureethylester gelöst. In einem anderen Glaskolben werden 20 ml Essigsäureethylester auf 45 °C erwärmt und dann unter Rühren beide Lösungen gleichzeitig zugetropft. Es bildet sich spontan eine starke Trübung. Nach Beendigung der Zugabe wird die Suspension noch 3 Stunden bei 50 °C gerührt. Anschliessend lässt man von selbst auf Raumtemperatur abkühlen. Der Niederschlag wird abfiltriert mit 10 ml Essigsäureethylester gewaschen und mittels Durchsaugen von Luft (5 Minuten) getrocknet. Man erhält 3,41 g (97,7% der Theorie) (+)-(1 R,2R)-3-[2-(Dimethylamino)methyl-(cyclohex-1-yl)]-phenol-maleat als kristalline Form A (gemäss Röntgen-Beugungsbild). Mittels DSC Analyse werden zwei Peaks bei 166,03 und 177,03 °C gefunden.

### Beispiel A6: Herstellung von kristallinem (+)-(1 R,2R)-3-[2-(Dimethylamino)methyl-(cyclohex-1-yl)]-phenol-maleat

### a) Herstellung

24,64 g (+)-(1R,2R)-3-[2-(Dimethylamino)methyl-(cyclohex-1-yl)]-phenol (Reinheit nach HPLC Analyse 76,8%) werden in einem 1I Dreihalskolben in 500 ml Essigsäureethylester gelöst. Man erwärmt auf 55 °C und gibt unter Rühren auf einmal 9,44 g Maleinsäure zu. Man rührt bei dieser Temperatur für 4 Stunden und dann für 2 Stunden bei 20 °C. Danach wird der Niederschlag abfiltriert, einmal mit 20 ml Essigsäureethylester gewaschen und anschliessend mittel Durchsaugen von Luft getrocknet. Bei der Thermoanalyse (DSC) werden endotherme Signale bei bei 150,4 °C und 157,7 °C gefunden. Die Reinheit (bezogen auf das Phenol) beträgt gemäss HPLC Analyse 84,4%. Die Ausbeute beträgt 27,57 g (97,0% der Theorie).

### b) Reinigung mittels Rührwaschens

Das so erhaltene Rohprodukt wird in Glaskolben eingewogen, mit Lösungsmittel versetzt und in einem Thermomixer bei 50 °C für 4 Stunden geschüttelt. Dann werden die Lösungen über Nacht auf 23 °C abgekühlt und danach filtriert. Die Ausbeute und die Reinheit (HPLC) des Rückstandes werden bestimmt. Weitere Angaben finden sich in nachfolgender Tabelle 1. Es werden folgende Abkürzungen verwendet: EtAc ist Ethylacetat, EtOH ist Ethanol, PrOH ist i-Propanol, AcNi ist Acetonitril, Tol ist Toluol, Hex ist n-Hexan, DiEt ist Diethylether, BuMe ist t-Butylmethylether, Ace ist Aceton, MeEt ist Methylethylketon, THF ist Tetrahydrofuran.

**Tabelle 1:**

| Menge Maleat (mg) | Lösungsmittel 1 (ml) | Lösungsmittel 2 (ml) | Ausbeute (%) | Reinheit (%) |
|---|---|---|---|---|
| 56,3 | EtAc (1,0) | -- | 97,2 | 84,6 |
| 55,9 | PrOH (1,0) | -- | 73,0 | 96,7 |
| 56,2 | Hex (1,0) | -- | 96,3 | 84,0 |
| 55,8 | AcNi (1,0) | -- | 71,5 | 93,6 |
| 56,4 | Tol (1,0) | -- | 97,2 | 84,0 |
| 62,6 | DiEt (1,0) | -- | 97,0 | 84,6 |
| 53,5 | BuMe (1,0) | -- | 97,4 | 84,2 |
| 55,2 | Ace (1,0) | -- | 58,2 | 97,9 |
| 55,1 | MeEt (1,0) | -- | 75,9 | 92,1 |
| 58,8 | THF (1,0) | -- | 80,1 | 89,5 |
| 55,2 | Ace (0,98) | EtOH (0,02) | 52,0 | 97,9 |
| 60,4 | Ace (0,96) | EtOH (0,04) | 50,5 | 97,0 |
| 55,5 | Ace (0,94) | EtOH (0,06) | 37,1 | 98,2 |
| 58,2 | Ace (0,92) | EtOH (0,08) | 42,4 | 98,3 |
| 57,3 | Ace (0,90) | EtOH (0,10) | 39,6 | 98,2 |
| 517,1 | PrOH( 10,0) | -- | 79,7 | 88,9 |
| 516,5 | PrOH( 10,0) | -- | 79,4 | 87,5 |
| 511,2 | PrOH(10,0) | -- | 80,7 | 89,0 |
| 516,4 | Ace (10,0) | -- | 62,0 | 98,1 |
| 515,2 | Ace (10,0) | -- | 64,8 | 96,4 |
| 511,6 | Ace (10,0) | -- | 68,7 | 94,8 |
| 506,9 | THF (10,0) | -- | 82,9 | 87,9 |
| 511,2 | THF (10,0) | -- | 81,9 | 87,8 |
| 507,2 | THF (10,0) | -- | 83,8 | 88,5 |
| 518,3 | Ace (9,70) | EtOH (0,3) | 48,3 | 98,6 |
| 514,3 | Ace (9,70) | EtOH (0,3) | 50,9 | 97,8 |
| 509,8 | Ace (9,70) | EtOH (0,3) | 50,2 | 98,7 |

Das Produkt kann durch Reinigung des Rohproduktes mittels Rührwaschen in guten bis exzellenten Reinheiten erhalten werden.

### B) Herstellung von amorphem (+)-(1 R,2R)-3-[2-(Dimethylamino)methyl-(cyclohex-1-yl)]-Phenol-maleat

### Beispiel B1: Gefriertrocknung

81,5 mg des gemäss Beispiel A1 hergestellten Maleats werden in 3 ml Wasser gelöst und dann auf -75 °C abgeschreckt. Dann wird bei dieser Temperatur und einem Druck von 0,011 mbar während 23 Stunden gefriergetrocknet. Man erhält quantitativ einen festen, weissen Rückstand, der in Gegenwart von Feuchtigkeit zur schnellen Kristallisation neigt, im wesentlichen unter Bildung der kristallinen Form A. Mittels Differential Scanning Calorimetrie (DSC, Erwärmungsrate 10 °C/Minute) wird eine Glasumwandlungstemperatur nahe 30 °C ermittelt. Ein exothermer Peak bei 85 °C wird auf eine Kristallisation zurückgeführt. Das gebildete kristalline Material schmilzt bei 178 °C.

Wenn man 20 mg der amorphen Form bei Raumtemperatur und 20% beziehungsweise 90% Luftfeuchtigkeit 24 Stunden lagert, erhält man eine Mischung der kristallinen Formen A und B beziehungsweise nur der kristallinen Form A.

### C) Herstellung der kristallinen Form A

Die Kristallform wird, wenn nicht anders angegeben, durch Vergleich der Raman-Spektren bestimmt.

### Beispiel C1: Fällung mit Nicht-Lösungsmittel

a) Fällung mit n-Heptan
   80 mg des gemäss Beispiel A1 hergestellten Maleats werden bei Raumtemperatur in 8 ml Aceton gelöst. Danach versetzt man unter Rühren mit 7.5 ml n-Heptan. Es bildet sich ein weisser Niederschlag, der abfiltriert und in einem Luftstrom bei Raumtemperatur getrocknet wird. Man erhält 50 mg weisses Pulver, das der kristallinen Form A entspricht.
b) Fällung mit n-Heptan
   80 mg des gemäss Beispiel A1 hergestellten Maleats werden bei Raumtemperatur in 17 ml Tetrahydrofuran (THF) gelöst. Danach versetzt man unter Rühren mit 9 ml n-Heptan. Es bildet sich ein weisser Niederschlag, der abfiltriert und in einem Luftstrom bei Raumtemperatur getrocknet wird. Man erhält 52 mg weisses Pulver, das der kristallinen Form A entspricht.
c) Fällung mit Teriärbutyl-methylether (TBME)
   80 mg des gemäss Beispiel A1 hergestellten Maleats werden bei Raumtemperatur in 21 ml THF gelöst. Danach versetzt man unter Rühren mit 7.5 ml TBME. Es bildet sich ein weisser Niederschlag, der abfiltriert und in einem Luftstrom bei Raumtemperatur getrocknet wird. Man erhält 48 mg weisses Pulver, das der kristallinen Form A entspricht.

### Beispiel C2: Phasengleichgewichte mit Suspensionen

a) Suspension in Aceton
   Zu 80 mg des gemäss Beispiel B1 hergestellten Maleats werden 0,5 ml Aceton zugegeben und man rührt 24 Stunden bei Raumtemperatur. Dann wird abfiltriert und in einem Luftstrom bei Raumtemperatur getrocknet. Man erhält 52 mg der kristallinen Form A als weisses Pulver.
b) Suspension in 1,4-Dioxan
   Zu 80 mg des gemäss Beispiel B1 hergestellten Maleats werden 0,5 ml 1,4-Dioxan zugegeben und man rührt 24 Stunden bei Raumtemperatur. Dann wird abfiltriert und in einem Luftstrom bei Raumtemperatur getrocknet. Man erhält 47 mg der kristallinen Form A als weisses Pulver.
c) Suspension in Ethylacetat
   Zu 80 mg des gemäss Beispiel B1 hergestellten Maleats werden 0,5 ml Ethylacetat zugegeben und man rührt 24 Stunden bei Raumtemperatur. Dann wird abfiltriert und in einem Luftstrom bei Raumtemperatur getrocknet. Man erhält 50 mg der kristallinen Form A als weisses Pulver.
d) Suspension in Wasser
   Zu 80 mg des gemäss Beispiel B1 hergestellten Maleats werden. 0,1 ml und danach zweimal je 0,2 ml Wasser zugegeben und man rührt 24 Stunden bei Raumtemperatur. Dann wird abfiltriert und in einem Luftstrom bei Raumtemperatur getrocknet. Man erhält 49 mg der kristallinen Form A als weisses Pulver.
e) Suspension in Isopropanol
   Zu 80 mg des gemäss Beispiel B1 hergestellten Maleats werden 0,5 ml Isopropanol zugegeben und man rührt 24 Stunden bei Raumtemperatur. Dann wird abfiltriert und in einem Luftstrom bei Raumtemperatur getrocknet. Man erhält 54 mg der kristallinen Form A als weisses Pulver.
f) Suspension in Tetrahydrofuran Zu 80 mg des gemäss Beispiel B1 hergestellten Maleats werden 0,5 ml THF zugegeben. Es bildet sich ein festes Agglomerat, das mit einem Spatel zerkleinert wird. Die erhaltene Suspension wird danach 24 Stunden bei Raumtemperatur gerührt. Dann wird abfiltriert und in einem Luftstrom bei Raumtemperatur getrocknet. Man erhält 49 mg der kristallinen Form A als weisses Pulver.

### Beispiel C3: Kristallisation

a) Kristallisation aus Methanol /Methylenchlorid
   100 mg des gemäss Beispiel A1 hergestellten Maleats werden in 1 ml Methanol gelöst. Nach Zugabe von 14 ml Methylenchlorid erhält man eine klare Lösung, die 5 Tage bei 5 °C stehen lässt. Dann wird das Volumen auf ein Viertel eingeengt und wieder bei 5 °C eine Woche lang stehen gelassen. Dann wird das Lösungsmittel bei Raumtemperatur vollständig verdampft. Man erhält 98 mg der kristallinen Form A in Form eines weissen Pulvers.
b) Kristallisation aus Toluol und Aceton
   60 mg des gemäss Beispiel A1 hergestellten Maleats werden in 10 ml Toluol / Aceton (1:1 v/v) bei 60 °C gelöst und danach auf Raumtemperatur abgekühlt. Man lässt dann die Lösung bei 5 °C für 4 Tage stehen. Danach werden die gebildeten, grossen Kristalle abfiltriert und getrocknet. Man erhält 28 mg der kristallinen Form A in Form weisser Kristalle.
c) Kristallisation aus Ethylacetat
   51 mg des gemäss Beispiel A1 hergestellten Maleats werden bei 65 °C in 31 ml Ethylacetat gelöst. Dann lässt man auf Raumtemperatur abkühlen und innerhalb von 2 Stunden bilden sich nadelförmige Kristalle. Man lässt noch 5 Tage bei 5 °C stehen, filtriert die Kristalle ab und trocknet. Man erhält 33 mg der kristallinen Form A in Form weisser, nadelförmiger Kristalle.
d) Kristallisation aus Tetrahydrofuran
   100 mg des gemäss Beispiel A1 hergestellten Maleats werden bei 65 °C in 7 ml THF gelöst und danach auf Raumtemperatur abgekühlt. Man lässt dann die Lösung bei 5 °C für 4 Tage stehen. Danach werden die gebildeten, grossen Kristalle abfiltriert und getrocknet. Man erhält 28 mg der kristallinen Form A in Form weisser, grosser Kristalle.
e) Kristallisation aus Isopropanol
   In einem Rührkolben werden 2,31 g des gemäss Beispiel A6a hergestellten Maleatsalzes in 30 ml Isopropanol suspendiert und innerhalb 20 Minuten bei einer Umdrehungszahl des Rührers von etwa 600 Upm (Umdrehungen pro Minute) auf 80 °C unter Bildung einer klaren Lösung erhitzt. Man erhöht die Umdrehungszahl auf 800 Upm und rührt bei dieser Temperatur für 30 Minuten, wobei gegen Ende die Umdrehungszahl kurz auf 1000 Upm erhöht wird. Dann wird innerhalb von 25 Minuten die Temperatur auf 65 °C gesenkt und 35 Minuten bei dieser Temperatur gerührt. Danach impft man mit 50 mg der kristallinen Form A des Maleatsalzes und an senkt unter Rühren innerhalb von 3 Stunden mit einer Rate von 0,3 K/min die Temperatur auf 10 °C. Der weisse Feststoff wird abfiltriert und an der Luft getrocknet (2,132 g, 92,3%). Gemäss Röntgen-Pulverdiffraktogramm werden nur Linien der kristallinen Form A gemessen. Die Thermoanalyse (DSC) zeigt endotherme Signale bei 165,8 °C und 177,2 °C. Bei der thermogravimetrischen Analyse wird kein signifikanter Gewichtsverlust beobachtet, so dass keine solvatisierte Form vorliegen kann. Die Reinheit beträgt, bestimmt mit HPLC, 99,5%.
f) Kristallisation aus Isopropanol
   In einem Rührkolben werden 2,32 g des gemäss Beispiel A6a hergestellten Maleatsalzes in 30 ml Isopropanol suspendiert und innerhalb 15 Minuten bei einer Umdrehungszahl des Rührers von etwa 600 Upm (Umdrehungen pro Minute) auf 80 °C unter Bildung einer klaren Lösung erhitzt. Man erhöht die Umdrehungszahl auf 800 Upm und rührt bei dieser Temperatur für 20 Minuten, wobei gegen Ende die Umdrehungszahl kurz auf 1000 Upm erhöht wird. Dann wird innerhalb von 10 Minuten die Temperatur auf 65 °C gesenkt und 10 Minuten bei dieser Temperatur gerührt. Danach impft man mit 50 mg der kristallinen Form A des Maleatsalzes an senkt unter Rühren innerhalb von 3 Stunden mit einer Rate von 0,25 K/min die Temperatur auf 20 °C. Der weisse Feststoff wird abfiltriert und an der Luft getrocknet (2,132 g, 92,3%). Gemäss Röntgen-Pulverdiffraktogramm werden nur Linien der kristallinen Form A gemessen. Die Thermoanalyse (DSC) zeigt endotherme Signale bei 163,4 °C und 174,4 °C. Bei der thermogravimetrischen Analyse wird kein signifikanter Gewichtsverlust beobachtet, so dass keine solvatisierte Form vorliegen kann. Die Reinheit beträgt, bestimmt mit HPLC, 99,5%.

### Beispiel C4: Kristallisation mittels Lösungsmittelverdampfung

a) Lösungsmittel Aceton / THF
   10 mg des gemäss Beispiel A1 hergestellten Maleats werden in 15 ml Aceton / THF (1:1, v/v) gelöst und das Lösungsmittelgemisch bei Raumtemperatur langsam im Stickstoffstrom bis zur Trockene verdampft. Man erhält 9 mg der kristallinen Form A in Form eines weissen Pulvers.
b) Lösungsmittel Aceton / Ethylacetat
   10 mg des gemäss Beispiel A1 hergestellten Maleats werden in 15 ml Aceton / Ethylacetat (1:1, v/v) gelöst und das Lösungsmittelgemisch bei Raumtemperatur langsam im Stickstoffstrom bis zur Trockene verdampft. Man erhält 8 mg der kristallinen Form A in Form eines weissen Pulvers.
c) Lösungsmittel Methylenchlorid
   10 mg des gemäss Beispiel A1 hergestellten Maleats werden in 15 ml Methylenchlorid (1:1, v/v) gelöst und das Lösungsmittelgemisch bei Raumtemperatur langsam unter Umgebungsbedingungen in offener Atmosphäre bei Raumtemperatur bis zur Trockene verdampft. Man erhält 9 mg der kristallinen Form A in Form eines weissen Pulvers.
d) Lösungsmittel THF / Wasser
   200 mg des gemäss Beispiel A1 hergestellten Maleats werden in 4 ml Wasser / THF (1:1, v/v) gelöst und die Lösung durch einen 0,22 µm Filter filtriert. Die Lösung wird auf eine Petrischale gegeben und das Lösungsmittelgemisch bei Raumtemperatur langsam unter Umgebungsbedingungen in offener Atmosphäre bei Raumtemperatur bis zur Trockene verdampft. Man erhält 188 mg der kristallinen Form A in Form nadelförmiger Kristalle.
e) Lösungsmittel THF / Wasser
   200 mg des gemäss Beispiel A1 hergestellten Maleats werden in 4 ml Wasser / THF (1:1, v/v) gelöst und die Lösung durch einen 0,22 µm Filter filtriert. Die Lösung wird auf eine Petrischale gegeben, mit Kristallen des Polymorphs B angeimpft und das Lösungsmittelgemisch bei Raumtemperatur langsam unter Umgebungsbedingungen in offener Atmosphäre bei Raumtemperatur bis zur Trockene verdampft. Nach einem Tag bilden sich nadelförmige Kristalle. Man erhält 160 mg der kristallinen Form A in Form nadelförmiger Kristalle.
f) Lösungsmittel THF / Wasser
   200 mg des gemäss Beispiel A1 hergestellten Maleats werden in 4 ml Wasser / THF (1:1, v/v) gelöst und die Lösung durch einen 0,22 µm Filter filtriert. Die Lösung wird auf eine Petrischale gegeben und das Lösungsmittelgemisch bei Raumtemperatur unter einem Stickstoffstrom bis zur Trockene verdampft. Nach einem Tag bilden sich nadelförmige Kristalle. Man erhält 182 mg der kristallinen Form A in Form nadelförmiger Kristalle.
g) Lösungsmittel 1,4-Dioxan
   11 mg des gemäss Beispiel A1 hergestellten Maleats werden in 15 ml 1,4-Dioxan gelöst. Die Lösung wird in ein offenes Fläschchen gegeben und das Lösungsmittel unter Umgebungsbedingungen in offener Atmosphäre bei Raumtemperatur innerhalb 4 Tagen bis zur Trockene verdampft. Man erhält 9 mg der kristallinen Form A in Form eines weissen Pulvers.
h) Lösungsmittel THF
   10 mg des gemäss Beispiel A1 hergestellten Maleats werden in 15 ml THF gelöst. Die Lösung wird auf eine Petrischale gegeben und das Lösungsmittelgemisch bei_ Raumtemperatur langsam unter Umgebungsbedingungen in offener Atmosphäre bei Raumtemperatur innerhalb 2 Tagen bis zur Trockene verdampft. Man erhält 9 mg der kristallinen Form A in Form eines weissen Pulvers.
i) Lösungsmittel THF / Wasser
   150 mg des gemäss Beispiel A1 hergestellten Maleats werden in 3 ml THF / Wasser (1:1, v/v) gelöst. Dann wird das Lösungmittel bei 60 °C am Rotationsverdampfer verdampft. Man erhält 135 mg der kristallinen Form A in Form eines weissen Pulvers.
j) Lösungsmittel THF / Wasser
   150 mg des gemäss Beispiel A1 hergestellten Maleats werden in 3 ml THF / Wasser (1:1, v/v) gelöst. Dann wird bei 40 °C 10 Minuten lang und darauf noch 1 Minute bei 60 °C gerührt. Man filtriert durch einen 0,22 µm Filter. Die Lösung wird auf eine Petrischale gegeben und das Lösungsmittelgemisch bei Raumtemperatur unter einem Stickstoffstrom bis zur Trockene verdampft. Man erhält 125 mg der kristallinen Form A in Form eines weissen Pulvers.
k) Lösungsmittel THF
   50 mg des gemäss Beispiel A1 hergestellten Maleats werden bei 60 °C in 40 ml THF gelöst. Dann wird mit Trockeneis gekühlt und bei -20 °C stehen gelassen, wobei sich eine Trübung bildet. Nach 3 Tagen hat sich ein weisser Niederschlag gebildet, der abfiltriert und getrocknet wird. Man erhält 21 mg der kristallinen Form A in Form eines weissen Pulvers.

### Beispiel C5: Umwandlung der kristallinen Form B in Form A

a) Phasengleichgewicht mit Suspension in Etylacetat
   30 mg der gemäss Beispiel D1 hergestellten kristallinen Form B werden in 2 ml Ethylacetat suspendiert und 18 Stunden bei 23 °C gerührt. Dann wird filtriert und der Rückstand getrocknet. Man erhält 15 mg der kristallinen Form A in Form eines weissen Pulvers.
b) Phasengleichgewicht mit Suspension in Toluol
   30 mg der gemäss Beispiel D1 hergestellten kristallinen Form B werden in 2 ml Toluol suspendiert und 18 Stunden bei 23 °C gerührt. Dann wird filtriert und der Rückstand getrocknet. Man erhält 22 mg der kristallinen Form A in Form eines weissen Pulvers.

### D) Herstellung der kristallinen Form B

### Beispiel D1:

250 mg des gemäss Beispiel A1 hergestellten Maleats werden in 5 ml THF / Wasser (1:1, v/v) gelöst. Dann wird bei 90 °C das Lösungsmittelgemisch am Rotationsverdampfer verdampft. Man erhält 208 mg der kristallinen Form B in Form eines weissen Pulvers. Der Schmelzpunkt beträgt etwa 178 °C und die Schmelzenthalpie beträgt etwa 125 J/g, gemessen mit DSC bei einer Aufheizrate von 10 °C/Minute. Das Röntgenbeugungsbild ist in Figur 3 und das Raman-Spektrum ist in Figur 4 dargestellt.

### Beispiel D2:

Es wird gemäss Beispiel D1 verfahren, aber vor Verdampfen des Lösungsmittel 22 Stunden stehen gelassen. Der Rückstand ist die kristalline Form B.

### Beispiel D3:

100 mg des gemäss Beispiel A1 hergestellten Maleats werden in 2 ml THF / Wasser (1:1, v/v) gelöst. Dann lässt man die Lösung 3,5 Stunden bei Raumtemperatur stehen. Danach wird bei 90 °C das Lösungsmittelgemisch am Rotationsverdampfer verdampft. Man erhält 84 mg der kristallinen Form B in Form eines weissen Pulvers.

### Beispiel D4:

Zweimal 250 mg des gemäss Beispiel A1 hergestellten Maleats werden in je 5 ml THF / Wasser (1:1, v/v) gelöst. Eine Lösung wird durch einen 0,22 µm Filter und die zweite Lösung durch einen 0,45 µm Filter filtriert. Die Lösungen werden auf je eine Petrischale gegeben und das Lösungsmittelgemisch bei Raumtemperatur langsam unter Umgebungsbedingungen in offener Atmosphäre bei Raumtemperatur verdampft. Nach einem Tag wird die Bildung von sphärischen Kristallen beobachten. Nach 3 Tagen ist das Lösungsmittelgemisch verdampft und die Rückstände bestehen aus der kristallinen Form B in Form von sphärischen Kristallen.

### E) Stabilitätsuntersuchungen

### Beispiel E1: Lagerung der amorphen Form in hoher Luftfeuchtigkeit

Die amorphe Form gemäss Beispiel B1 wird bei Raumtemperatur und 75% relativer Luftfeuchtigkeit gelagert und nach 2 Tagen, 1, 2, 3, 4 und 8 Wochen werden Proben mittels Raman Spektroskopie untersucht. Nach 2 Tagen liegt eine Mischung der kristallinen Formen A und B vor. Die gleiche Mischung wird auch noch nach 8 Wochen beobachtet.

### Beispiel E2: Lagerung der kristallinen Form A in hoher Luftfeuchtigkeit

Das gemäss Beispiel A1 hergestellte Maleat wird bei Raumtemperatur und 75% relativer Luftfeuchtigkeit gelagert und nach 3 Tagen, 1, 2, 3, 4, 8 und 10 Wochen werden Proben mittels Raman Spektroskopie untersucht. Die Proben sind auch nach 10 Wochen praktisch unverändert.

### Beispiel E3: Lagerung der kristallinen Form B in hoher Luftfeuchtigkeit

Die gemäss Beispiel D1 hergestellte kristalline Form B wird bei Raumtemperatur und 75% relativer Luftfeuchtigkeit gelagert und nach 3 Tagen, 1, 2, 3, 4 und 8 Wochen werden Proben mittels Raman Spektroskopie untersucht. Die Proben sind auch nach 8 Wochen praktisch unverändert.

### Beispiel E4: Stabilität unter Malbedingungen

a) Kristalline Form A beim Verreiben
   Das gemäss Beispiel A1 hergestellte Maleat wird in einer Mörserschale mit einem Stössel 5 Minuten lang verrieben. Danach liegt unverändert die kristalline Form A vor. Amorphe Anteile werden nicht beobachtet.
b) Kristalline Form B beim Verreiben
   Die kristalline Form B gemäss Beispiel D1 wird in einer Mörserschale mit einem Stössel 5 Minuten lang verrieben. Danach liegt eine Mischung der kristallinen Form A und B vor.
c) Kristalline Form A beim Mahlen
   Das gemäss Beispiel A1 hergestellte Maleat wird in eine Achat-Kugelmühle gefüllt (Retsch MM200 Mischmühle mit 5 mm Achatkugel) und bei 20 Hz und Raumtemperatur 180 Minuten gemahlen. Danach liegt unverändert die kristalline Form A vor. Amorphe Anteile werden nicht beobachtet.
c) Kristalline Form B beim Mahlen
   Mit der kristallinen Form B gemäss Beispiel D1 wird gemäss Beispiel E4c) verfahren. Nach 180 Minuten liegt ein Gemisch der kristallinen Formen A und B vor.

### Beispiel E5: Stabilität unter Druck

a) Kristalline Form A beim Presen von Tabletten
   Das gemäss Beispiel A1 hergestellte Maleat wird in eine Tablettenpresse gefüllt und unter Vakuum bei einem Druck von 100 MPa für 60 Minuten zu einer Tablette gepresst. Danach liegt unverändert die kristalline Form A vor.
b) Kristalline Form B beim Presen von Tabletten
   Mit der kristallinen Form B gemäss Beispiel D1 wird gemäss Beispiel E5a) verfahren. Nach 60 Minuten liegt ein Gemisch der kristallinen Formen A und B vor.

### Beispiel E6: Wasseraufnahme

Die Wasseraufnahme wird mittels dynamischer Wasserdampfaufnahme (Dynamic Vapor Sorption, DVS) mit dem Gerät DVS-1 der Firma Surface Measurement Systems Ltd.ermittelt. Die Probe wird in einem Platintiegel an der Spitze einer Mikrowaage plaziert. Dann wird die Probe zunächst bei 50% relativer Luftfeuchtigkeit äquilibriert und dann einem vordefinierten Messprogramm unterzogen. Die Temperatur beträgt 25 °C. Bestimmt wird die Gewichtsänderung der Probe.
a) Amorphe Form
   Die amorphe Form weist bis etwa 33% relative Luftfeuchtigkeit eine ausgeprägte Wasseraufnahme von etwa 2,2 Gew.-% auf. Bei höherer Luftfeuchtigkeit fällt das Gewicht wieder ab, was durch eine Kristallisation verursacht wird. Nach Ende des Versuchs liegt ein Mischung der kristallinen Formen A und B vor.
b) Kristalline Form A
   Die kristalline Form A weist eine Wasseraufnahme von lediglich etwa 0,2 Gew.-%. Am Ende des Messzyklus liegt im wesentlichen die unveränderte kristalline Form A vor.
c) Kristalline Form B
   Die kristalline Form B weist eine Wasseraufnahme von lediglich etwa 0,2 Gew.-% auf. Am Ende des Messzyklus liegt jedoch ein Gemisch der kristallinen Formen A und B vor.

### Beispiel E7: Stabilität bei erhöhter Temperatur

Die kristallinen Formen werden in offenen und geschlossen Gefässen gelagert und nach einer vorgegebenen Zeit mögliche Veränderungen chromatographisch (HPLC) als Stabilitätsparameter gemessen. Die kristalline Form A erweist sich hinsichtlich chemischer Stabilität und Kristallform als sehr stabil. Die kristalline Form B erweist sich hinsichtlich chemischer Stabilität als sehr stabil. Die Ergebnisse sind in der nachfolgenden Tabelle 2 angegeben.

**Tabelle 2:**

| Polymorp h | Bedingungen | Zeit | HPLC | FT Raman¹⁾ |
|---|---|---|---|---|
| A | 40 °C/75% r.L. | 1 Woche | 100,2% | A |
| A | 60 °C/75% r.L. | 4 Wochen | 100,3% | A |
| A | 60 °C (geschlossen) | 1 Woche | 100,2% | A |
| A | 60 °C (geschlossen) | 4 Wochen | 100,5% | A |
| A | -18 °C (Referenz) | 4 Wochen | 100% | A |
| B | 40 °C/75% r.L. | 1 Woche | 100.3% | A und B |
| B | 60 °C/75% r.L. | 4 Wochen | 101,1% | A und B |
| B | 60 °C (geschlossen) | 1 Woche | 100,6% | A und B |
| B | 60 °C (geschlossen) | 4 Wochen | 100,9% | A und B |
| B | -18 °C (Referenz) | 4 Wochen | 100% | A und B |

| | | | | |
|---|---|---|---|---|
| ¹⁾ Bestimmung kristalline Form | | | | |

### Beispiel E8: Wasseraufnahme von Maleat und Hydrochlorid (Vergleich)

Die Wasseraufnahme wird mittels dynamischer Wasserdampfaufnahme (Dynamic Vapor Sorption, DVS) mit dem Gerät DVS-1 der Firma Surface Measurement Systems Ltd. Ermittelt. Die Probe wird in einem Platintiegel an der Spitze einer Mikrowaage plaziert. Dann wird die Probe zunächst bei 50% relativer Luftfeuchtigkeit äquilibriert und dann einem vordefinierten Messprogramm unterzogen. Die Temperatur beträgt 25 °C. Bestimmt wird die Gewichtsänderung der Probe nach einer schrittweisen Erhöhung der relativen Luftfeuchtigkeit um je 10% bis auf 90%.
a) (+)-(1 R.2R)-3-[2-(Dimethylamino)methyl-(cyclohex-1-yl)1-phenol-maleat (Form A) Es wird eine ausgesprochen geringe Wasseraufnahme von 0,06% gefunden. Die absorbierte Feuchte wird bei etwa 10% relativer Luftfeuchtigkeit wieder vollständig abgegeben.
b) (+)-(1R,2R)-3-[2-(Dimethylamino)methyl-(cyclohex-1-yl)]-phenol-Hydrochlorid (Form D)
   Bei einer Erhöhung der relativen Luftfeuchtigkeit bis etwa 75% wird eine Wasseraufnahme von etwa 2 Gew.-% beobachtet. Eine weitere Erhöhung bis 90% relativer Luftfeuchtigkeit führt zu einer Wasseraufnahme von gesamt etwa 5 Gew.-%. Bei der Desorption durch schrittweise Erniedrigung der relativen Luftfeuchtigkeit um 10% wird mehr Wasser abgegeben als zuvor aufgenommen, nämlich insgesamt 8,5%. Es wird vermutet, dass ein variables Hydrat vorliegt.

Beispiel E9: Feuchtlagerung und Trocknung von Maleat und Hydrochlorid (Vergleich) Maleat und Hydrochlorid gemäss Beispiel E8 werden zunächst bei 25 °C und 75% und 95% relativer Luftfeuchtigkeit 7 Tage gelagert und danach 7 Tage im Trockenschrank bei 50 °C im Vakuum getrocknet. Vor Beginn der Lagerung werden Röntgenbeugungsbilder (XRD), DSC zur Bestimmung von Übergangspunkten (Tp), des Trockengewichts (TG) und der Wassergehalt nach Karl-Fischer (WG) bestimmt und die gleichen Werte nach der Feuchtlagerung und Trocknung. Die Röntgenbeugungsbilder zeigen keine Veränderung der kristallinen Formen A und D nach Feuchtlagerung und Trocknung. Die Ausgangswerte für das Hydrochlorid sind Tp = 116,27 °C, TG = 6,35% und WG 8,00%. Die Ausgangswerte für das Maleat sind Tp = 170,4 °C und 179.85 °C, TG = 0 und WG = 0,2%. Die Ergebnisse sind in Tabellen 3 (75% relative Luftfeuchtigkeit) und 4 (95% relative Luftfeuchtigkeit) angegeben.

**Tabelle 3 (75%):**

| Salz | Tp (°C) | TG (%) | WG (%) | Tp (°C) | TG (%) | WG (%) |
|---|---|---|---|---|---|---|
| Maleat | 165.41 | -- | 0,20 | 165,33 | - | 0,20 |
| Hydrochlori d | 116,22 | 5,92 | 8,50 | 113,83 | 6,12 | 6,00 |

**Tabelle 4 (95%):**

| Salz | Tp (°C) | TG (%) | WG (%) | Tp (°C) | TG | WG (%) |
|---|---|---|---|---|---|---|
| Maleat | 165.92 177,82 | -- | 0,20 | 165,47 177,55 | -- | 0,50 |
| Hydrochlori d | 116,52 | 7,84 | 8,50 | 114,47 | 6,74 | 6,60 |

### Geräte, Methoden:

Differential Scanning Calorimetrie (DSC): Gerätebezeichnung Perkin Elmer DSC 7 oder Perkin Elmer Pyris 1. Variable Messungen (Heizungsrate) in Gold- oder Aluminiumtiegeln.

### Pulver Röntgenstrahlung Beugungsbilder (PXRD):

PXRD wird mit einem Philips 1710 Pulver X-Strahlung Diffraktometer durchgeführt, wobei CuK_{α}-Strahlung verwendet wird. D-Abstände werden von den 2θ Werten berechnet, wobei die Wellenlänge von 1.54060 Å zu Grunde gelegt ist. Es gilt allgemein, dass die 2θ Werte eine Fehlerrate von ±0.1-0.2° besitzen. Der experimentelle Fehler bei den d-Abstandswerten ist daher abhängig vom Ort der Linie (des Peaks).

### Raman Spektroskopie:

FT-Raman Spektren werden mit einem Bruker RFS 100 FT-Raman System aufgenommen, der mit einem Nd:YAG Laser (Wellenlänge 1064 nm) und einem mit flüssigen Stickstoff gekühltem Germanium Detektor betrieben wird. Für jede Probe werden 64 Abtastungen mit einer Auflösung von 2 cm⁻¹ akkumuliert. Generell wird eine Laserleistung von 100 mW verwendet.

### Beschreibung der Figuren

Figur 1 zeigt das Röntgen-Beugungsbild der polymorphen Form A
Figur 2 zeigt das Raman-Spektrum der polymorphen Form A
Figur 3 zeigt das Röntgen-Beugungsbild der polymorphen Form B
Figur 4 zeigt das Raman-Spektrum der polymorphen Form B

## Patentansprüche

1. Salze von Maleinsäure und 3-[2-(Dimethylamino)methyl-(cyclohex-1-yl)]-phenol, der Formel I

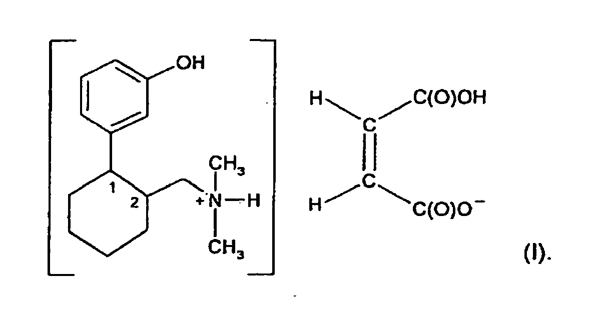

2. Salze gemäss Anspruch 1, **dadurch gekennzeichnet, dass** die Verbindung der Formel I als Diastereomer oder Gemisch von enantiomeren Diastereomeren mit Transkonfiguration des Phenylringes und der Dimethylaminomethylgruppe (1R,2R-beziehungsweise 1S,2S-Konfiguration) vorliegt.

3. Salze gemäß Anspruch 1 oder 2, wobei die Verbindung als Enantiomer mit der absoluten Konfiguration (1 R, 2R) vorliegt.

4. Verfahren zur Herstellung von 3-[2-(Dimethylamino)methyl-(cyclohex-1-yl)]-phenol-maleat der Formel I gemäß Anspruch 1-3 umfassend die Vereinigung der Komponenten 3-[2-(Dimethytamino)methyl-(cyclohex-1-yl)]-phenol und Maleinsäure.

5. Verfahren gemäß Anspruch 4 zur Herstellung von 3-[2-(Dimethylamino)methyl-(cyclohex-1-yl)]-phenol-maleat der Formel I gemäß Anspruch 1, umfassend die Schritte
a) Lösen von 3-[2-(Dimethylamino)methyl-(cyclohex-1-yl)]-phenol in einem Lösungsmittel,
b) Vermischen der Lösung mit Maleinsäure oder einer Lösung von Maleinsäure, und
c) Isolieren der Verbindung der Formel I.

6. Salz gemäß Anspruch 1-3, wobei das Salz in der kristallinen Form A vorliegt, die ein charakteristisches Röntgen-Beugungsbild im Bereich von 2° bis 35° 2θ mit ausgeprägten charakteristischen Linien aufweist, ausgedrückt in d-Werten (Å): 9.4 (vs), 6.8 (m), 5.56 (s), 5.30 (s), 5.22 (s), 4.71 (s), 4.66 (s), 4.24 (m), 4.12 (m), 4.03 (m), 3.98 (s) 3.76 (m), 3.27 (m).

7. Salz gemäß Anspruch 1-3, wobei das Salz in der kristallinen Form A vorliegt, charakterisiert durch ein Pulverdiffraktogramm umfassend einen oder mehrere der folgenden Reflexe: 9,38, 9,94 und 10,35 (jeweils ± 0,5 in 2 Theta).

8. Salz gemäß Anspruch 7, wobei das Salz in der kristallinen Form A vorliegt,
**dadurch gekennzeichnet, dass** das Pulverdiagramm zusätzlich einen oder mehrere der folgenden Reflexe aufweist: 12,76, 15,94, 17,54, 19,28, 28,68 und 31,99 (jeweils ± 0,2 in 2 Theta).

9. Salz gemäß Anspruch 1-3, wobei das Salz in der kristallinen Form A vorliegt, charakterisiert durch ein Raman-Spektrum umfassend eine oder mehrere der folgenden Banden, jeweils ausgedrückt in Wellenzahlen (cm-1): 118 (vs), 188 (w), 400 (w), 676 (w), 2812 (w), 2879 (m).

10. Salz gemäß Anspruch 9, charakterisiert durch eine oder mehrere der folgenden Banden: 118 (vs), 188 (w), 292 (m), 328 (m), 359 (w), 400 (w), 486 (vw), 676 (w), 901 (w),1025 (w), 1273 (m), 1351 (m), 1412 (w), 1569 (vw), 1601 (m),1690 (m), 2812 (w), 2879 (m), 2986 (m), 3060 (m).

11. Salz, wobei das Salz in der kristallinen Form A vorliegt , gemäß einem oder mehreren der Ansprüche 6-10, **dadurch gekennzeichnet, dass** 3-[2-(Dimethylamino)methyl-(cyclohex-1-yl)]-phenol-maleat in der Konfiguration (1R, 2R) vorliegt.

12. Salz gemäß Anspruch 1-3, wobei das Salz in der kristallinen Form B vorliegt, die ein charakteristisches Röntgen-Beugungsbild im Bereich von 2° bis 35° 2θ mit ausgeprägten charakteristischen Linien aufweist, ausgedrückt in d-Werten (Å): 10.6 (m), 7.5 (m), 7.3 (m), 6.1 (s), 5.29 (s) 4.88 (m), 4.72 (m), 4.47 (vs), 4.43 (m), 4.26 (m), 4.24 (m), 3.99 (s), 3.71 (m), 3.52 (m), 3.30 (s).

13. Salz gemäß Anspruch 1-3, wobei das Salz in der kristallinen Form B vorliegt, charakterisiert durch ein Pulverdiffraktogramm umfassend einen oder mehrere der folgenden Reflexe: 8,36, 14,5 und 14,83 (jeweils ± 0,5 in 2 Theta).

14. Salz gemäß Anspruch 13, wobei das Salz in der kristallinen Form B vorliegt,
**dadurch gekennzeichnet, dass** das Pulverdiagramm zusätzlich einen oder mehrere der folgenden Reflexe aufweist:
11,85, 12,19, 18,16, 22,85, 29,1 und 29,41 (jeweils ± 0,2 in 2 Theta.

15. Salz gemäß Anspruch 1-3, wobei das Salz in der kristallinen Form B vorliegt, charakterisiert durch ein Raman-Spektrum umfassend eine oder mehrere der folgenden Banden, jeweils ausgedrückt in Wellenzahlen (cm-1): 229 (m), 875 (m) und 2829 (m).

16. Salz, wobei das Salz in der kristalline Form B vorliegend, gemäß Anspruch 15. charakterisiert durch eine oder mehrere der folgenden Banden: 229 (m), 307 (w), 372 (w), 605 (vw), 875 (m), 890 (w), 1010 (w), 1197 (m), 1401 (vw), 1480 (vw), 1583 (m), 1703 (s), 2829 (m), 2953 (s).

17. Verfahren zur Herstellung der kristallinen Form B von 3-[2-(Dimethylamino)methyl-(cyclohex-1-yl)]-phenol-maleat, **dadurch gekennzeichnet, dass** man 3-[2-(Dimethylamino)methyl-(cyclohex-1-yl)]-phenol-maleat in einer Mischung aus Tetrahydrofuran und Wasser im Volumenverhältnis von 0,8:1,2 bis 1,2:0,8 löst, und dann das Lösungsmittelgemisch (a) bei einer Temperatur von mindestens 80 °C, oder (b) bei Raumtemperatur in einem offenen Gefäss in offener Atmosphäre im Kontakt mit Luftfeuchtigkeit vollständig entfernt, oder (c) eine Suspension von 3-[2-(Dimethylamino)methyl-(cyclohex-1-yl)]-phenol-maleat in einem Lösungsmittel als Träger bei einer Temperatur von mindestens 120 °C bis zur vollständigen Bildung der kristallinen Form B rührt.

18. Salz, wobei das Salz in der kristallinen Form B vorliegt, erhältlich nach einem Verfahren gemäß Anspruch 17.

19. Salz, wobei das Salz in der kristallinen Form B vorliegt gemäß einem oder mehreren der Ansprüche 12-16 und 18, **dadurch gekennzeichnet, dass** 3-[2-(Dimethylamino)methyl-(cyclohex-1-yl)]-phenol-maleat in der Konfiguration (1 R, 2R) vorliegt.

20. Pharmazeutische Zusammensetzung enthaltend eine wirksame Menge 3-[2-(Dimethylamino)methyl-(cyclohex-1-yl)]-phenol-maleat der Formel I gemäss Anspruch 1-19 und einen pharmazeutischen Träger oder ein pharmazeutisches Verdünnungsmittel.

21. Zusammensetzung gemäss Anspruch 20, worin die Verbindung der Formel I als kristalline Form A, kristalline Form B oder in einer Mischung der Formen A und B vorliegt.

22. Zusammensetzung gemäss Anspruch 21, worin die Verbindung der Formel I als kristalline Form A enthalten ist.

23. Verwendung von 3-[2-(Dimethylamino)methyl-(cyclohex-1-yl)]-phenol-maleat der Formel I gemäss Anspruch 1-22 zur Herstellung einer pharmazeutischen Zusammensetzung zur Behandlung von Schmerz.

24. 3-[2-(Dimethylamino)methyl-(cyclohex-1-yl)]-phenol-maleat der Formel I gemäß Anspruch 1-22 zur Behandlung von Schwerz.

## Claims

1. Salts of maleic acid and 3-[2-(dimethylamino)methyl-(cyclohex-1-yl)]-phenol of the formula I

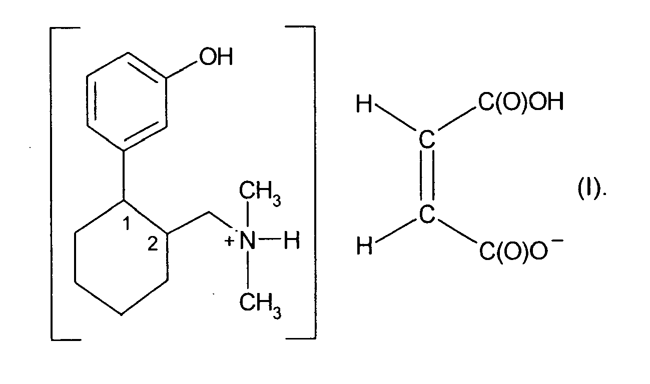

2. Salts according to claim 1, **characterised in that** the compound of the formula I is present as a diastereomer or a mixture of enantiomeric diastereomers with transconfiguration of the phenyl ring and of the dimethylaminomethyl group (1 R,2R or 1 S,2S configuration).

3. Salts according to claim 1 or 2, the compound being present as an enantiomer with the absolute configuration (1 R,2R).

4. A method for the production of 3-[2-(dimethylamino)methyl-(cyclohex-1-yl)]-phenol maleate of the formula I according to claim 1-3 comprising the combination of the components 3-[2-(dimethylamino)methyl-(cyclohex-1-yl)]-phenol and maleic acid.

5. A method according to claim 4 for the production of 3-[2-(dimethylamino)-methyl-(cyclohex-1-yl)]-phenol maleate of the formula I according to claim 1, comprising the steps
a) dissolving 3-[2-(dimethylamino)methyl-(cyclohex-1-yl)]-phenol in a solvent,
b) mixing the solution with maleic acid or a solution of maleic acid, and
c) isolating the compound of the formula I.

6. A salt according to claim 1-3, the salt being present as crystalline form A, which comprises a characteristic X-ray diffraction pattern in the range from 2° to 35° 2θ with pronounced characteristic lines, expressed in d values (Å):
9.4 (vs), 6.8 (m), 5.56 (s), 5.30 (s), 5.22 (s), 4.71 (s), 4.66 (s), 4.24 (m), 4.12 (m), 4.03 (m), 3.98 (s) 3.76 (m), 3.27 (m).

7. A salt according to claim 1-3, the salt being present as crystalline form A, **characterised by** a powder diffractogram comprising one or more of the following reflections: 9.38, 9.94 and 10.35 (in each case ± 0.5 in 2 theta).

8. A salt according to claim 7, the salt being present as crystalline form A, **characterised in that** the powder diagram additionally comprises one or more of the following reflections: 12.76, 15.94, 17.54, 19.28, 28.68 and 31.99 (in each case ± 0.2 in 2 theta).

9. A salt according to claim 1-3, the salt being present as crystalline form A, **characterised by** a Raman spectrum comprising one or more of the following bands, in each case expressed in wavenumbers (cm-1): 118 (vs), 188 (w), 400 (w), 676 (w), 2812 (w), 2879 (m).

10. A salt according to claim 9, **characterised by** one or more of the following bands:
118 (vs), 188 (w), 292 (m), 328 (m), 359 (w), 400 (w), 486 (vw), 676 (w), 901 (w), 1025 (w), 1273 (m), 1351 (m), 1412 (w), 1569 (vw), 1601 (m), 1690 (m), 2812 (w), 2879 (m), 2986 (m), 3060 (m).

11. A salt, the salt being present as crystalline form A, according to one or more of claims 6-10, **characterised in that** 3-[2-(dimethytamino)methyl-(cyclohex-1-yl)]-phenol maleate is present in the configuration (1 R,2R).

12. A salt according to claim 1-3, the salt being present as crystalline form B, which comprises a characteristic X-ray diffraction pattern in the range from 2° to 35° 2θ with pronounced characteristic lines, expressed in d values (Å): 10.6 (m), 7.5 (m), 7.3 (m), 6.1 (s), 5.29 (s) 4.88 (m), 4.72 (m), 4.47 (vs), 4.43 (m), 4.26 (m), 4.24 (m), 3.99 (s), 3.71 (m), 3.52 (m), 3.30 (s).

13. A salt according to claim 1-3, the salt being present as crystalline form B, **characterised by** a powder diffractogram comprising one or more of the following reflections: 8.36, 14.5 and 14.83 (in each case ± 0.5 in 2 theta).

14. A salt according to claim 13, the salt being present as crystalline form B, **characterised in that** the powder diagram additionally comprises one or more of the following reflections:
11.85, 12.19, 18.16, 22.85, 29.1 and 29.41 (in each case ± 0.2 in 2 theta).

15. A salt according to claim 1-3, the salt being present as crystalline form B, **characterised by** a Raman spectrum comprising one or more of the following bands, in each case expressed in wavenumbers (cm-1): 229 (m), 875 (m) and 2829 (m).

16. A salt, the salt being present as crystalline form B, according to claim 15, **characterised by** one or more of the following bands:
229 (m), 307 (w), 372 (w), 605 (vw), 875 (m), 890 (w), 1010 (w), 1197 (m), 1401 (vw), 1480 (vw), 1583 (m), 1703 (s), 2829 (m), 2953 (s).

17. A method for the production of the crystalline form B of 3-[2-(dimethylamino)-methyl-(cyclohex-1-yl)]-phenol maleate, **characterised in that** 3-[2-(dimethylamino)methyl-(cyclohex-1-yl)]-phenol maleate is dissolved in a mixture of tetrahydrofuran and water in the volume ratio of 0.8:1.2 to 1.2:0.8, and then the solvent mixture is removed completely (a) at a temperature of at least 80°C, or (b) at room temperature in an open vessel in an open atmosphere in contact with atmospheric humidity, or (c) a suspension of 3-[2-(dimethylamino)methyl-(cyclohex-1-yl)]-phenol maleate is stirred in a solvent as carrier at a temperature of at least 120°C until formation of the crystalline form B is complete.

18. A salt, the salt being present as crystalline form B, obtainable by a method according to claim 17.

19. A salt, the salt being present as crystalline form B, according to one or more of claims 12-16 and 18, **characterised in that** 3-[2-(dimethylamino)methyl-(cyclohex-1-yl)]-phenol maleate is present in the configuration (1R,2R).

20. A pharmaceutical composition containing an active quantity of 3-[2-(dimethylamino)methyl-(cyclohex-1-yl)]-phenol maleate of the formula I according to claim 1-19 and a pharmaceutical excipient or a pharmaceutical diluent.

21. A composition according to claim 20, in which the compound of the formula I is present as crystalline form A, crystalline form B or in a mixture of forms A and B.

22. A composition according to claim 21, in which the compound of the formula I is contained as crystalline form A.

23. Use of 3-[2-(dimethylamino)methyl-(cyclohex-1-yl)]-phenol maleate of the formula I according to claim 1-22 to produce a pharmaceutical composition for treating pain.

24. 3-[2-(Dimethylamino)methyl-(cyclohex-1-yl)]-phenol maleate of the formula I according to claim 1-22 for treating pain.

## Revendications

1. Sels d'acide maléique et de 3-[2-(diméthylamino)-méthyl-(cyclohex-1-yl)]-phénol de formule I

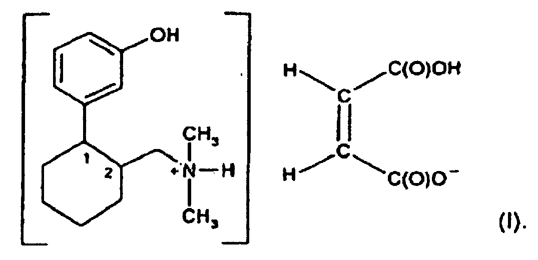

2. Sels suivant la revendication 1, **caractérisés en ce que** le composé de formule I se présente comme diastéréoisomère ou comme mélange de diastéréoisomères énantiomères avec la configuration trans du noyau phényle et du groupe diméthylaminométhyle (respectivement configuration 1R,2R et configuration 1S,2S).

3. Sels suivant la revendication 1 ou 2, le composé se présentant sous forme d'énantiomère ayant la configuration absolue (1R, 2R) .

4. Procédé de production de maléate de 3- [2- (diméthylamino)méthyl-(cyclohex-1-yl)]-phénol de formule I suivant les revendications 1-3, comprenant la mise en présence des composants 3-[2-(diméthylamino)méthyl-(cyclohex-1-yl)]-phénol et acide maléique.

5. Procédé suivant la revendication 4, pour la production de maléate de 3-[2-(diméthylamino)méthyl-(cyclohex-1-yl)]-phénol de formule I suivant la revendication 1, comprenant les étapes de
a) dissolution de 3-[2-(diméthylamino)méthyl-(cyclohex-1-yl)]-phénol dans un solvant,
b) mélange de la solution avec de l'acide maléique ou avec une solution d'acide maléique,
et
c) isolement du composé de formule I.

6. Sel suivant les revendications 1-3, le sel se présentant sous la forme cristalline A ayant un diagramme de diffraction des rayons X caractéristique dans la plage de valeurs 2θ de 2° à 35° avec des raies caractéristiques accentuées, exprimées en valeurs d (en Å) : 9,4 (très forte), 6,8 (moyenne), 5,56 (forte), 5,30 (forte), 5,22 (forte), 4,71 (forte), 4,66 (forte), 4,24 (moyenne), 4, 12 (moyenne), 4,03 (moyenne), 3,98 (forte), 3,76 (moyenne), 3,27 (moyenne).

7. Sel suivant les revendications 1-3, le sel se présentant sous la forme cristalline A, **caractérisé par** un diagramme de diffraction de poudre comprenant une ou plusieurs des réflexions suivantes : 9,38, 9,94 et 10,35 (à chaque fois ± 0,5 en 2 thêta).

8. Sel suivant la revendication 7, le sel se présentant sous la forme cristalline A, **caractérisé par le fait que** le diagramme de diffraction de poudre comprend en outre une ou plusieurs des réflexions suivantes : 12,76, 15,94, 17,54, 19,28, 28,68 et 31,99 (à chaque fois ± 0,2 en 2 thêta).

9. Sel suivant les revendications 1-3, le sel se présentant sous la forme cristalline A, **caractérisé par** un spectre Raman comprenant une ou plusieurs des raies suivantes, exprimées dans chaque cas en nombres d'ondes (cm⁻¹) : 118 (très forte), 188 (faible), 400 (faible), 676 (faible), 2812 (faible), 2879 (moyenne).

10. Sel suivant la revendication 9, **caractérisé par** une ou plusieurs des raies suivantes : 118 (très forte), 188 (faible), 292 (moyenne), 328 (moyenne), 359 (faible), 400 (faible), 486 (très faible), 676 (faible), 901 (faible), 1025 (faible), 1273 (moyenne), 1351 (moyenne), 1412 (faible), 1569 (très faible), 1601 (moyenne), 1690 (moyenne), 2812 (faible), 2879 (moyenne), 2986 (moyenne), 3060 (moyenne).

11. Sel, le sel se présentant sous la forme cristalline A, selon une ou plusieurs des revendications 6-10, **caractérisé en ce que** le maléate de 3-[2-(diméthylamino)méthyl-(cyclohex-1-yl)]-phénol est présent avec la configuration (1R,2R).

12. Sel selon les revendications 1-3, le sel étant sous la forme cristalline B, qui présente une image de diffraction des rayons X caractéristique dans la plage de valeurs 2θ de 2° à 35° avec des raies caractéristiques accentuées, exprimées en valeurs d (en Å): 10,6 (moyenne), 7,5 (moyenne, 7,3 (moyenne), 6,1 (forte), 5,29 (forte), 4,88 (moyenne), 4,72 (moyenne), 4,47 (très forte), 4,43 (moyenne), 4,26 (moyenne), 4,24 (moyenne), 3,99 (forte), 3,71 (moyenne), 3,52 (moyenne), 3,30 (forte).

13. Sel suivant les revendications 1-3, le sel étant sous la forme cristalline B, **caractérisé par** un diagramme de diffraction de poudre comprenant une ou plusieurs des réflexions suivantes 8,36, 14,5 et 14,83 (à chaque fois ± 0,5 en 2 thêta).

14. Sel suivant la revendication 13, le sel étant sous la forme cristalline B, **caractérisé par** un diagramme de diffraction de poudre présentant en outre une ou plusieurs des réflexions suivantes : 11,85, 12,19, 18,16, 22,85, 29,1 et 29,41 (à chaque fois ± 0,2 en 2 thêta).

15. Sel suivant les revendications 1-3, le sel étant sous la forme cristalline B, **caractérisé par** un spectre Raman comprenant une ou plusieurs des raies suivantes, exprimées dans chaque cas en nombres d'ondes (cm⁻¹) : 229 (moyenne), 875 (moyenne) et 2829 (moyenne).

16. Sel, le sel se présentant sous la forme cristalline B, suivant la revendication 15, **caractérisé par** une ou plusieurs des bandes suivantes : 229 (moyenne), 307 (faible), 372 (faible), 605 (très faible), 875 (moyenne), 890 (faible), 1010 (faible), 1197 (moyenne), 1401 (très faible), 1480 (très faible), 1583 (moyenne), 1703 (forte), 2829 (moyenne), 2953 (forte).

17. Procédé de production de la forme cristalline B du maléate de 3-[2-(diméthylamino)méthyl-(cyclohex-1-yl)]-phénol, **caractérisé en ce qu'**on dissout du maléate de 3-[2-(diméthylamino)méthyl-(cyclohex-1-yl)]-phénol dans un mélange de tétrahydrofuranne et d'eau dans un rapport en volume de 0,8:1,2 à 1,2:0,8, puis on chasse complètement le mélange de solvants (a) à une température d'au moins 80°C ou (b) à la température ambiante dans un récipient ouvert en atmosphère ouverte au contact de l'humidité de l'air, ou bien (c) on agite une suspension de maléate de 3-[2-(diméthylamino)méthyl-(cyclohex-1-yl)]-phénol dans un solvant utilisé comme véhicule à une température d'au moins 120°C jusqu'à l'obtention complète de la forme cristalline B.

18. Sel, le sel se présentant sous la forme cristalline B, pouvant être obtenu par un procédé selon la revendication 17.

19. Sel, le sel se présentant sous la forme cristalline B selon une ou plusieurs des revendications 12-16 et 18, **caractérisé en ce que** maléate de 3- [2- (diméthylamino) - méthyl-(cyclohex-1-yl)]-phénol présente la configuration (1R,2R).

20. Composition pharmaceutique contenant une quantité efficace de maléate de 3-[2-(diméthylamino)méthyl-(cyclohex-1-yl) ] -phénol de formule I suivant les revendications 1-19 et un support pharmaceutique ou un diluant pharmaceutique.

21. Composition suivant la revendication 20, dans laquelle le composé de formule I est présent sous la forme cristalline A, sous la forme cristalline B, ou en un mélange des formes A et B.

22. Composition suivant la revendication 21, dans laquelle le composé de formule I est contenu sous la forme cristalline A.

23. Utilisation du maléate de 3-[2-(diméthylamino)-méthyl-(cyclohex-1-yl)]-phénol de formule I suivant les revendications 1-22 pour la préparation d'une composition pharmaceutique destinée au traitement de la douleur.

24. Maléate de 3-[2-(diméthylamino)méthyl-(cyclohex-1-yl) ] -phénol de formule I suivant les revendications 1-22 destiné au traitement de la douleur.
